(19) Europäisches Patentamt
European Patent Office
Office européen des brevets

(11) EP 4 538 382 A1

(12) EUROPEAN PATENT APPLICATION

(43) Date of publication:
**16.04.2025 Bulletin 2025/16**

(21) Application number: **23202738.3**

(22) Date of filing: **10.10.2023**

(51) International Patent Classification (IPC):
***C12P 5/00*** *(2006.01)* ***C12N 1/20*** *(2006.01)*
***C12N 9/02*** *(2006.01)* ***C12N 9/90*** *(2006.01)*
***C12P 23/00*** *(2006.01)*

(52) Cooperative Patent Classification (CPC):
**C12P 23/00; C12N 1/20; C12N 9/0077;**
**C12N 9/0083; C12N 9/90; C12P 5/007;**
C12Y 114/99; C12Y 505/01019

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Faculty of Biology & CeBiTec Universität Bielefeld**
**33615 Bielefeld (DE)**

(72) Inventors:
- **MEYER, Florian**
  **33604 Bielefeld (DE)**
- **WENDISCH, Volker**
  **33617 Bielefeld (DE)**
- **HENKE, Nadja Alina**
  **32139 Spenge (DE)**

(74) Representative: **Eisenführ Speiser Patentanwälte Rechtsanwälte PartGmbB**
**Gollierstraße 4**
**80339 München (DE)**

Remarks:
The complete document including Reference Table(s) and the Sequence Listing(s) can be downloaded from the EPO website

# (54) CAROTENOID PRODUCTION

(57) The present invention relates to a method for producing one or more species of carotenoids via one or more producer organism(s). The present invention also relates to a growth medium comprising ammonium sulfate, urea, phosphate, glucose, and potassium acetate, for production of one or more species of carotenoids via a producer organism. The present invention also relates to a kit comprising growth medium and one or more producer organism(s). The present invention also relates to o the use of an expression construct and a cell in a method according to the present invention.

EP 4 538 382 A1

## Description

## Technical Field

The present invention relates to a method for producing one or more species of carotenoids via one or more producer organism(s). The present invention also relates to a growth medium comprising ammonium sulfate, urea, phosphate, glucose, and potassium acetate, for production of one or more species of carotenoids via a producer organism. The present invention also relates to a kit comprising growth medium and one or more producer organism(s). The present invention also relates to o the use of an expression construct and a cell in a method according to the present invention.

## Background of the Invention

Carotenoids are yellow, orange, and red organic pigments that are produced by plants and algae, as well as some bacteria and fungi. For example, carotenoids provide the characteristic color to carrots, pumpkins, parsnips, corn, tomatoes, salmon, lobster, and shrimp. All carotenoids are derivatives of terpenes. Currently, there are more than 1,100 identified carotenoids, which can be subdivided into two groups: (i) xanthophylls (which contain oxygen) and (ii) carotenes (which are hydrocarbons and do not contain oxygen).

Belonging to the group of xanthophylls, astaxanthin is a keto-carotenoid, which is a metabolite of zeaxanthin and canthaxanthin, containing both hydroxyl and ketone functional groups. Astaxanthin is a hydrophobic pigment with red coloring properties, resulting from the extended chain of conjugated double bonds at the center of the compound.

Naturally, astaxanthin is for example produced by the yeast *Xanthophyllomyces dendrorhous* (also known as *Phaffia rhodozyma*) and the fresh water microalgae *Haematococcus pluvialis* under stress conditions, such as nutrient deficiency, increased salinity, or excess sunlight. Thus, animals feeding on the algae, such as different species of fish like salmon, red trout, and red sea bream, or different crustaceans reflect the typical red-orange astaxanthin pigmentation. Said animals are incapable of synthesizing astaxanthin themselves. Therefore, in aquaculture, astaxanthin has to be added to the feed to gain intense flesh coloring.

Moreover, astaxanthin is used as a dietary supplement for humans as well as in the cosmetics industry, for example in anti-aging products.

Industrial astaxanthin production via chemical synthesis requires petrochemical sources. Between 95% and 99% of commercially available astaxanthin is produced this way. The remainder is obtained via extraction of astaxanthin producing microorganisms, mostly from the afore-mentioned *Phaffia rhodozyma* or *Haematococcus pluvialis.* The cost for chemically synthesized astaxanthin is about 1,000 USD per kg, whereas the cost for extracted natural astaxanthin is much higher ranging from about 2,500 to 7,500 USD per kg.

Astaxanthin exists in three different configurational isomers: two enantiomers (3'S 3S and 3'R 3R) and in meso form (3'S 3R). Among these different configurations, the 3'S 3S form is superior in regards to its antioxidant properties. Whereas natural astaxanthin is typically found in the (preferred) 3'S 3S form, chemically synthesized astaxanthin usually contains a mixture of the three different forms 3'S 3S, 3'R 3R, and 3'S 3R. Consequently, there is a growing demand for natural astaxanthin. However, extraction of natural astaxanthin is subject to seasonal variation and cannot easily be scaled to meet growing demand among customers for natural products from renewable and green sources.

In order to meet the globally growing demand for natural astaxanthin scalable biotechnological approaches for astaxanthin production are highly sought after. Hence, research to establishing new producer organisms has gained momentum. Among the newly identified producer organisms, *Corynebacterium glutamicum* (*C. glutamicum*) was found to be especially promising.

*C. glutamicum* is a Gram-positive, rod-shaped bacterium, which has been safely used for more than 50 years for the industrial production of amino acids, such as glutamate and lysine at the million ton scale. Mutants of *C. glutamicum* have also been identified that produce various other amino acids. Thus, *C. glutamicum* is an established production host in the food and feed industries that grows to high cell densities with high growth rates. Moreover, the physiology of C. *glutamicum* is very well understood with an elaborated genetic tool box being readily available. C. *glutamicum* naturally produces the carotenoid decaprenoxanthin. Both astaxanthin and decaprenoxanthin derive from the common precursor molecule lycopene. Hence, metabolic engineering efforts were invested in order to use *C. glutamicum* for the production of other carotenoids, such as astaxanthin.

US 2009/0221027 discloses that a heterologous metI overexpression in connection with the disruption of a MarR-type regulator (denoted as CrtR herein) may be used for production of methionine and carotenoids, but provides no further information, especially on the production of lysine and astaxanthin.

WO 2018/197608 A1 discloses a method for producing astaxanthin and lysine in recombinant Gram-positive bacteria comprising a nucleic acid sequence encoding for a CrtZ-protein from *Fulvimarina pelagi* and comprises a nucleic acid sequence encoding for a CrtW enzyme.

However, to date no systematic optimization of growth medium for C. *glutamicum* intended for use as a producer

organism for carotenoids has been performed. The importance of such an optimization became apparent upon observing that already an addition of 2 % (w/v) of potassium acetate to the medium (CGXII medium) can lead to an increase in astaxanthin content of about 83 % from 1.7 mg/g cell dry weight (CDW) to 3.11 mg /g CDW. CGXII medium contains high concentrations of nitrogen sources, as it was originally developed for production of lysine, which is an amino acid with two ammonium groups. Thus, further medium optimization for the production of carotenoids, especially astaxanthin, was envisioned as part of the present invention.

It was thus an object of the present invention to provide a mineral salt medium, which is optimized for energy efficient production of carotenoids via a producer organism.

It was a particular object of the present invention to provide a mineral salt medium, which is optimized for energy efficient production of astaxanthin via a producer organism.

It was a further object of the present invention to provide a mineral salt medium, which is optimized for cost efficient production of carotenoids via a producer organism.

It was a particular object of the present invention to provide a mineral salt medium, which is optimized for cost efficient production of astaxanthin via a producer organism.

It was also an object of the present invention to provide a mineral salt medium, which is optimized for time efficient production of carotenoids via a producer organism.

It was also an object of the present invention to provide a mineral salt medium, which is optimized for time efficient production of astaxanthin via a producer organism.

It was also an object of the present invention to provide a mineral salt medium, which is optimized for sustainable production of carotenoids via a producer organism.

It was also an object of the present invention to provide a mineral salt medium, which is optimized for sustainable production of astaxanthin via a producer organism.

It was also an object of the present invention to provide a method for energy efficiently producing carotenoids via a producer organism using the mineral salt medium according to the present invention.

It was also an object of the present invention to provide a method for energy efficiently producing astaxanthin via a producer organism using the mineral salt medium according to the present invention.

It was also an object of the present invention to provide a method for energy efficiently producing astaxanthin in combination with one or more further carotenoids via a producer organism using the mineral salt medium according to the present invention.

It was also an object of the present invention to provide a method for cost efficiently producing carotenoids via a producer organism using the mineral salt medium according to the present invention.

It was also an object of the present invention to provide a method for cost efficiently producing astaxanthin via a producer organism using the mineral salt medium according to the present invention.

It was also an object of the present invention to provide a method for cost efficiently producing astaxanthin in combination with one or more further carotenoids via a producer organism using the mineral salt medium according to the present invention.

It was also an object of the present invention to provide a method for time efficiently producing carotenoids via a producer organism using the mineral salt medium according to the present invention.

It was also an object of the present invention to provide a method for time efficiently producing astaxanthin via a producer organism using the mineral salt medium according to the present invention.

It was also an object of the present invention to provide a method for time efficiently producing astaxanthin in combination with one or more further carotenoids via a producer organism using the mineral salt medium according to the present invention.

It was also an object of the present invention to provide a method for sustainably producing carotenoids via a producer organism using the mineral salt medium according to the present invention.

It was also an object of the present invention to provide a method for sustainably producing astaxanthin via a producer organism using the mineral salt medium according to the present invention.

It was also an object of the present invention to provide a method for sustainably producing astaxanthin in combination with one or more further carotenoids via a producer organism using the mineral salt medium according to the present invention.

It was also an object of the present invention to provide a use of the mineral salt medium according to the present invention for energy efficient production of carotenoids via a producer organism.

It was also an object of the present invention to provide a use of the mineral salt medium according to the present invention for cost efficient production of carotenoids via a producer organism.

It was also an object of the present invention to provide a use of the mineral salt medium according to the present invention for time efficient production of carotenoids via a producer organism.

It was also an object of the present invention to provide a use of the mineral salt medium according to the present invention for sustainable production of carotenoids via a producer organism.

It was also an object of the present invention to provide a use of the mineral salt medium according to the present invention for energy efficient production of astaxanthin via a producer organism.

It was also an object of the present invention to provide a use of the mineral salt medium according to the present invention for cost efficient production of astaxanthin via a producer organism.

It was also an object of the present invention to provide a use of the mineral salt medium according to the present invention for time efficient production of astaxanthin via a producer organism.

It was also an object of the present invention to provide a use of the mineral salt medium according to the present invention for sustainable production of astaxanthin via a producer organism.

**Description of Figures**

**Figure 1** Contour plots of the response surface model for the effects of the main media components of medium A on astaxanthin titers. Astaxanthin titers are given as mg/L and all other concentrations as g/L. The response surface is sliced at Block = 1.47, Glucose = 40.67, Acetate = 9.67, Ammonium sulfate = 19, Urea = 2.58, Phosphate = 3.55. A change in shading from dark to light indicates an increasing titer of astaxanthin.

**Figure 2** Contour plots of the RSM for the five trace metals and their effect on astaxanthin titers. All concentrations are given as mg/L and the astaxanthin titers are shown as labels in the plots. The model is based on a five factor RSM, performed in a BioLector cultivation system using strain *Corynebacterium glutamicum* ASTA* at 30°C and 1100 rpm. A change in shading from dark to light indicates an increasing titer of astaxanthin.

**Figure 3** BioLector experiment to show the effects of the optimization of the medium A base medium and its trace metals on carotenoid formation (left y-axis) and cell dry weight (CDW; right y-axis). All cells were grown in 1 ml of medium at 1100 rpm and 30°C for 48 h. The strain used was *Corynebacterium glutamicum* ASTA*. The medium line indicates the composition of the base medium, meaning Glucose, Potassium acetate, Ammonium sulfate, Urea and Phosphate concentrations while the Trace metals line indicates either the normal medium A trace metal composition or the optimized composition derived in this study as described in Example 6.

**Description of Sequences**

| SEQ ID NO: | Sequence description |
|---|---|
| 1 | Amino acid sequence of lycopene cyclase enzyme CrtY |
| 2 | Amino acid sequence of β-carotene ketolase enzyme CrtW |
| 3 | Amino acid sequence of β-carotene hydroxylase enzyme CrtZ |
| 4 | Nucleic acid sequence of lycopene cyclase gene *crtY* (DNA) |
| 5 | Nucleic acid sequence of β-carotene ketolase gene *crtW* (DNA) |
| 6 | Nucleic acid sequence of β-carotene hydroxylase gene *crtZ* (DNA) |
| 7 | Ribosomal binding site: GAAAGGAGG |
| 8 | Ribosomal binding site: GAAAGAAGG |
| 9 | Ribosomal binding site: GAATGGAGG |
| 10 | Ribosomal binding site: GAATGAAGG |
| 11 | Spacing sequence: CCCTGGAG |
| 12 | Spacing sequence: CCCTGG |
| 13 | Spacing sequence: CCC |
| 14 | Nucleic acid sequence of MarR-type regulator gene CrtR (DNA) |
| 15 | Amino acid sequence of MarR-type regulator CrtR |
| 16 | Nucleic acid sequence of16S rRNA molecule of *Corynebacterium glutamicum* (DNA) |

(continued)

| SEQ ID NO: | Sequence description |
|---|---|
| 17 | Nucleic acid sequence of16S rRNA molecule of *Corynebacterium glutamicum* (RNA) |
| 18 | Nucleic acid sequenc of $P_{tuf}$ promoter (DNA) |

Whenever the present disclosure relates to the percentage of identity of nucleic acid or amino acid sequences to each other these values define those values as obtained by using the EMBOSS Water Pairwise Sequence Alignments (nucleotide) program or the EMBOSS Water Pairwise Sequence Alignments (protein) program for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see Smith, T.F. & Waterman, M.S. "identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a polypeptide can be "codon-optimized" if the respective sequence is to be used in another organism in comparison to the original organism a molecule originates from.

It is preferred that the terms "sequence identity" and "sequence similarity" may be used as synonyms, i.e. interchangeably. Thus, it is preferred that the term "sequence similarity" may refer to the sequence identity.

**Detailed Description of the Invention**

In a first aspect, the present invention relates to a method for producing one or more species of carotenoids via one or more producer organism(s) comprising the steps of

(i) Providing a growth medium comprising ammonium sulfate, urea, phosphate, glucose, and acetate,

wherein the concentration ratio of glucose to potassium acetate in said medium is in the range of from 3 .0 to 4.0 and

the concentration ratio of glucose to ammonium sulfate in said medium is in the range of from 2.0 to 5.5 and

the concentration ratio of glucose to urea in said medium is in the range of from 6.0 to 12.5 and

the concentration ratio of glucose to phosphate in said medium is in the range of from 11.0 to 27.0;

(ii) Adding one or more producer organism(s) to said growth medium;

(iii) Cultivating said one or more producer organism(s) in said growth medium, under conditions allowing production of one or more species of carotenoids;

(iv) Optionally: extracting the one or more species of carotenoids.

The term carotenoids as used herein refers to yellow, orange, and red organic pigments that are produced by photosynthetic organisms, such as plants or algae, as well as several bacteria, archaea, and/or fungi. Carotenoids are typically unsaturated with conjugated double-bonds to a high degree and thus absorb wavelengths ranging from 400 to 550 nanometers (violet to green light). In nature, Carotenoids mainly function as accessory pigments to chlorophyll in the light-harvesting part of photosynthesis. Most carotenoids are isoprenoids. Carotenoids may comprise modifications, which can be selected from the non-limiting group consisting of cyclization, varying degrees of saturation or unsaturation, and the presence of one or more functional group(s). Typically, carotenoids are hydrocarbons, i.e. only contain hydrogen and carbon. However, carotenoids may also contain oxygen. Oxygen containing carotenoids are known as xanthophylls, wherein the group of xanthophylls includes lutein, zeaxanthin, β-cryptoxanthin, 3'-hydroxyechinenone, cantahrxanthin, adonixanthin, adonirubin, decaprenoxanthin and astaxanthin. Carotenoids are lipophilic due to the presence of long aliphatic chains.

Preferably, the term "species of carotenoids" as used herein may be used interchangeably with the term carotenoid(s).

The term "producer organism" as used herein refers to any organism, which is capable of synthesizing one or more species of carotenoids. Producer organisms can be prokaryotic or eukaryotic organisms. Producer organisms can be prokaryotic organisms, such as bacteria and/or archaea. Producer organisms can be eukaryotic organisms, such as fungi, algae and/or plants. Producer organisms can naturally harbor all or part of the necessary enzymes required for the synthesis of one or more species of carotenoids. Producer organisms can alternatively harbor all or part of the necessary enzymes required for the synthesis of one or more species of carotenoids as result of genetic manipulation, such as metabolic engineering.

The term "growth medium" as used herein may refer to a liquid, solid, or semi-solid designed to support the growth of a population of microorganisms, especially bacteria, archaea, and fungi, or eukaryotic cells via the process of cell proliferation. The term "growth medium" as used herein may refer to media used for cell culture, which use specific cell types derived from plants or animals, as well as to those used for microbiological culture, which are used for growing microorganisms such as bacteria, archaea, or fungi. The term "growth medium" as used herein may refer to different types of media, which can be selected from the non-limiting group consisting of culture media, minimal media, selective media, and differential media. The term "growth medium" as used herein may also refer to enriched media. The term "growth medium" as used herein may referto both undefined media (also known as complex media) and defined media. In undefined media comprise a source of amino acids and nitrogen, wherein the source of amino acids consists of a variety of different compounds. In defined media, all contained chemicals are known and can specifically be defined as no yeast, animal or plant extract or tissue has been added during the preparation process.

The term "culture medium" as used herein preferably refers to growth media, which contain all elements that most microorganisms, especially most bacteria, archaea, and fungi, need for growth and which are not selective.

The term "minimal medium" as used herein refers to defined media having just enough ingredients to support growth of a given microorganism, especially bacteria, archaea, or fungi, or of a given eukaryotic cell. The number of ingredients as well as the amount of each respective ingredient added to a minimal medium varies depending on which microorganism or which eukaryotic cell is being grown or cultivated. Mineral media may or may not contain one or more source(s) of amino acid(s) depending on which microorganism or which eukaryotic cell is being grown or cultivated. Mineral media comprise one or more component(s) selected from the group consisting of one or more carbon source(s), one or more salt(s), and water. The term "mineral medium" as used herein also encompasses supplementary mineral media, which comprise at least one additional component allowing for the cultivation of auxotrophic microorganisms, especially specific lines of auxotrophic recombinants.

The term "selective medium" as used herein refers to growth media only allowing for growth of selected microorganisms, especially selected bacteria, archaea, or fungi, or selected eukaryotic cells. Selective media preferably comprise one or more substance(s) suitable for allowing selection of the one or more microorganism(s) and/or one or more eukaryotic cell(s) based on one or more properties, such as for example reporter gene(s), preferably one or more selectable marker(s). The one or more substance(s) suitable for allowing selection of the one or more microorganism(s) and/or one or more eukaryotic cell(s) can for example be selected from the non-limiting group consisting of antibiotic agents, antiviral agents, and small organic molecules, such as sugars.

The term "differential medium" as used herein refers to growth media, which allow distinguishing one or more type(s) of microorganism(s) from one or more other type(s) of microorganism(s) and/or one or more type(s) of eukaryotic cell(s) from one or more other type(s) of eukaryotic cell(s) growing in the presence of one or more specific nutrient(s) and/or one or more specific indicator(s). Such indicators are added to the medium to visibly indicate the defining characteristics of a microorganism and/or eukaryotic cell. Such indicators can be selected from the non-limiting group consisting of neutral red, phenol red, eosin y, or methylene blue. Differential media allow for the detection of specific microorganisms and/or specific eukaryotic cells, particularly of recombinant strains of microorganisms. The terms "differential medium" and "indicator medium" as used herein can be used interchangeably.

The term "growth" as used herein preferably refers to cell proliferation, wherein the term "cell proliferation" refers to cell growth in terms of size as well as to the division (e.g. binary fission or budding) of cells into daughter cells. In the context of microorganisms, especially prokaryotic microorganisms, the term "growth" as used herein particularly refers to cell division through binary fission and/or budding. In the context of eukaryotic organisms or eukaryotic cells, the term "growth" as used herein particularly refers to the entire process of cell proliferation including meiosis and mitosis as well as to the entire process of cell proliferation including amitosis, particularly in the context of fungi and protista.

The term "microorganism" as used herein preferably refers to organisms of microscopic scale, wherein microscopic scale is the scale of objects and events smaller than those that can easily be seen by the naked eye. The term "microorganism" as used herein preferably refers to organisms with a diameter of 10 $\mu$m or less, wherein the diameter is determined at the widest position in case a given microorganism is of non-circular shape. The term "microorganism" as used herein preferably refers to prokaryotic organisms, such as bacteria and archaea. The term "microorganism" as used herein preferably also refers to eukaryotic organisms of microscopic scale, particularly fungi, plants, algae, and protista.

In a preferred embodiment of the method according to the present invention, the one or more species of

carotenoids produced are one or more xanthophylls.

In a preferred embodiment of the method according to the present invention, the, one, two, three or more or all species of carotenoids produced are xanthophylls selected from the group consisting of lutein, zeaxanthin, β-cryptoxanthin, 3'-hydroxyechinenone, cantahrxanthin, adonixanthin, adonirubin, decaprenoxanthin and astaxanthin.

In a preferred embodiment of the method according to the present invention, the or one species of carotenoids produced is lutein.

In a preferred embodiment of the method according to the present invention, the or one species of carotenoids produced is zeaxanthin.

In a preferred embodiment of the method according to the present invention, the or one species of carotenoids produced is β-cryptoxanthin.

In a preferred embodiment of the method according to the present invention, the or one species of carotenoids produced is 3'-hydroxyechinenone.

In a preferred embodiment of the method according to the present invention, the or one species of carotenoids produced is cantahrxanthin.

In a preferred embodiment of the method according to the present invention, the or one species of carotenoids produced is adonixanthin.

In a preferred embodiment of the method according to the present invention, the or one species of carotenoids produced is adonirubin.

In a preferred embodiment of the method according to the present invention, the or one species of carotenoids produced is decaprenoxanthin.

In a preferred embodiment of the method according to the present invention, the or one species of carotenoids produced is astaxanthin.

In a preferred embodiment of the method according to the present invention, the one or more species of carotenoids produced is astaxanthin in combination with one or more xanthophylls selected from the group consisting of lutein, zeaxanthin, β-cryptoxanthin, 3'-hydroxyechinenone, cantahrxanthin, adonixanthin, adonirubin, and decaprenoxanthin.

The term "ammonium sulfate" as used herein preferably refers to an inorganic salt with the CAS Number 7783-20-2. The term "ammonium sulfate" as used herein preferably refers to an inorganic salt with the chemical formula $(NH_4)_2SO_4$. The term "ammonium sulfate" as used herein preferably refers to ammonium sulfate, ammonium sulfate (2:1), diammonium sulfate, sulfuric acid diammonium salt, mascagnite, actamaster, or dolamin. Accordingly, the term "ammonium sulfate" as used herein and the terms "ammonium sulfate (2:1)", "diammonium sulfate", "sulfuric acid diammonium salt", "mascagnite", "actamaster", and "dolamin" can be used interchangeably. The term "ammonium sulfate" as used herein preferably refers to an inorganic salt with a structure and composition according to formula (I).

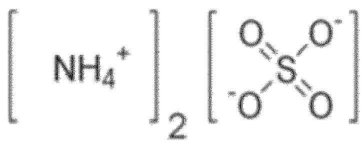

(I)

The term "sulfate" as used herein preferably refers to a polyatomic anion with the CAS Number 14808-79-8. The term "sulfate" as used herein preferably refers to a polyatomic anion with an empirical formula according to formula (II), wherein the sulfate anion consists of a central sulfur atom surrounded by four equivalent oxygen atoms in a tetrahedral arrangement.

$$SO_4^{2-} \quad \text{(II)}$$

The term "sulfate" as used herein preferably refers to sulfate, tetraoxosulfate(VI), and/or tetraoxidosulfate(VI). Accordingly, the term "sulfate" as used herein and the terms "tetraoxosulfate(VI)", and "tetraoxidosulfate(VI)" can be used interchangeably.

The term "urea" as used herein preferably refers to a chemical compound with the CAS Number 57-13-6. The term "urea" as used herein preferably refers to a chemical compound with the chemical formula $CO(NH_2)_2$, wherein the amide has two amino groups ($-NH_2$) joined by a carbonyl functional group ($-C(=O)-$). The term "urea" as used herein refers to a chemical compound with a structure according to formula (III) .

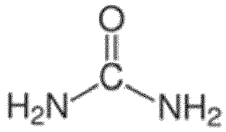

(III)

The term "urea" as used herein preferably refers to carbamide, carbonyldiamide, carbonyldiamine, diaminomethanal, and/or diaminomethanone. Accordingly, the term "urea" as used herein and the terms "carbamide", "carbonyldiamide", "carbonyldiamine", "diaminomethanal", and "diaminomethanone" can be used interchangeably.

The term "phosphate" as used herein preferably refers to an anion with the CAS Number 14265-44-2 derived from phosphoric acid. The term "phosphate" as used herein preferably refers to an anion with the empirical formula according to formula (IV), wherein a central phosphorus atom surrounded by four oxygen atoms in a tetrahedral arrangement.

$$[PO_4]^{3-} \qquad (IV)$$

The term "phosphate" as used herein preferably refers to orthophosphate, tetraoxophosphate(V), and/or tetraoxidophosphate(V). Accordingly, the term "phosphate" as used herein and the terms "orthophosphate", "tetraoxophosphate(V)", and "tetraoxidophosphate(V)" can be used interchangeably.

The term "glucose" as used herein preferably refers to a monosaccharide, i.e. a hexose, with the molecular formula $C_6H_{12}O_6$ and a CAS Number of 50-99-7. The term "glucose" as used herein preferably refers to both the open-chain (acyclic) as well as the ring (cyclic) form of the glucose molecule. The term "glucose" as used herein preferably refers to the D-isomer, D-glucose, also known as dextrose as well as to the L-isomer, L-glucose. The term "glucose" as used herein preferably refers to D-gluco-hexose, (2R,3S,4R,5R)-2,3,4,5,6-pentahydroxyhexanal, (3R,4S,5S,6R)-6-(hydroxymethyl)oxane-2,3,4,5-tetrol, dextrose, corn sugar, and grape sugar. Accordingly, it is preferred that the term "glucose" and the terms "D-gluco-hexose", "(2R,3S,4R,5R)-2,3,4,5,6-pentahydroxyhexanal", "(3R,4S,5S,6R)-6-(hydroxymethyl) oxane-2,3,4,5-tetrol", "dextrose", "corn sugar", and "grape sugar" can be used interchangeably.

The term "acetate" as used herein preferably refers to the conjugate base or ion (specifically, the negatively charged ion called an anion), which is part of salt formed by the combination of acetic acid with a base and typically found in aqueous solution and written with the chemical formula according to formula (V).

$$C_2H_3O_2^- \qquad (V)$$

The skilled person knows how to calculate and adapt values given for the concentration or the absolute amount of ions to the respective concentration or absolute amount of any salt of said ion.

The term "concentration ratio" as used herein preferably refers to the ratio of volume-based concentrations of two compounds, which are present in the same composition. The concentration ratio can be based on the mass (or weight) of a given compound in a given volume of a composition. Alternatively, the concentration ratio can be based on the number of molecules of a given compound in a given volume of a composition. For determining the concentration ratio, the volume-based concentration of a first compound is divided by the volume-based concentration of a second compound, wherein for both compounds the same unit of measurement denoting the volume-based concentration of said compound is used. Typical units of measurement denoting volume-based concentrations are known to persons skilled in the art. For example, if the concentration ratio of compound A to compound B is determined to be 2, the respective composition contains twice as much mass (or weight) of compound A as compared to compound B in a given volume of the composition in case the volume-based concentrations of compounds A and B are given in mass (or weight) per volume. For example, if a composition comprises compound A at a concentration of 100 g/L and compound B at a concentration of 50 g/L, the concentration ratio of compound A to compound B would be 2. Alternatively, if the concentration ratio of compound A to compound B is determined to be 2, the respective composition contains twice as many parts (e.g. molecules) of compound A as compared to compound B in a given volume of the composition in case the volume-based concentrations of compounds A and B are given in parts (e.g. molecules) per volume. For example, if a composition comprises compound A at a concentration of 2 M and compound B at a concentration of 1 M, the concentration ratio of compound A to compound B would be 2. How to convert units of measurements denoting volume-based concentrations based on mass (or weight) per volume into units of measurements denoting volume-based concentrations based on parts (e.g. molecules) per volume is known to a person skilled in the art. Preferably, the term "concentration ratio" refers to the mass ratio and these terms may be used interchangeably. Preferably, the term "concentration ratio" refers to the molar ratio and these terms may be used interchangeably.

Preferably in the method according to the present invention, the concentration ratio of glucose to potassium acetate in the growth medium provided in step (i) is in the range of from 3.1 to 4, preferably in the range of from 3.2 to 4,

preferably in the range of from 3.3 to 4, preferably in the range of from 3.4 to 4, preferably in the range of from 3.5 to 4, preferably in the range of from 3.6 to 4, preferably in the range of from 3.7 to 4, preferably in the range of from 3.8 to 4, particularly preferably in the range of from 3.9 to 4.

Preferably in the method according to the present invention, the concentration ratio of glucose to potassium acetate in the growth medium provided in step (i) is in the range of from 3.1 to 4, preferably in the range of from 3.2 to 4, preferably in the range of from 3.3 to 4, preferably in the range of from 3.4 to 4, preferably in the range of from 3.5 to 4, preferably in the range of from 3.6 to 4, preferably in the range of from 3.7 to 4, preferably in the range of from 3.8 to 4, particularly preferably in the range of from 3.9 to 4, wherein the concentration ratio of glucose to potassium acetate is calculated based on mass (or weight) per volume of glucose and potassium acetate.

Preferably in the method according to the present invention, the concentration ratio of glucose to potassium acetate in the growth medium provided in step (i) is in the range of from 3.1 to 3.9, preferably in the range of from 3.2 to 3.8, preferably in the range of from 3.3 to 3.7, preferably in the range of from 3.4 to 3.6, particularly preferably in the range of from 3.5 to 3.6.

Preferably in the method according to the present invention, the concentration ratio of glucose to potassium acetate in the growth medium provided in step (i) is in the range of from 3.1 to 3.9, preferably in the range of from 3.2 to 3.8, preferably in the range of from 3.3 to 3.7, preferably in the range of from 3.4 to 3.6, particularly preferably in the range of from 3.5 to 3.6, wherein the concentration ratio of glucose to potassium acetate is calculated based on mass (or weight) per volume of glucose and potassium acetate.

Preferably in the method according to the present invention, the concentration ratio of glucose to potassium acetate in the growth medium provided in step (i) is 3.94, wherein the concentration ratio of glucose to potassium acetate is calculated based on mass (or weight) per volume of glucose and potassium acetate.

Preferably in the method according to the present invention, the concentration ratio of glucose to potassium acetate in the growth medium provided in step (i) is 3.52, wherein the concentration ratio of glucose to potassium acetate is calculated based on mass (or weight) per volume of glucose and potassium acetate.

Preferably in the method according to the present invention, the concentration of glucose in the growth medium provided in step (i) is in the range of from 30 g/L to 70 g/L, preferably of from 35 g/L to 65 g/L, preferably of from 40 g/L to 60 g/L, preferably of from 40 g/L to 55 g/L, preferably of from 40 g/L to 50 g/L, particularly preferably of from 42 g/L to 50 g/L.

Preferably in the method according to the present invention, the concentration of glucose in the growth medium provided in step (i) is 42.6 g/L.

Preferably in the method according to the present invention, the concentration of glucose in the growth medium provided in step (i) is 50 g/L.

Preferably in the method according to the present invention, the concentration of potassium acetate in the growth medium provided in step (i) is in the range of from 8 g/L to 15 g/L, preferably of from 9 g/L to 14 g/L, preferably of from 10 g/L to 13 g/L, preferably of from 11 g/L to 13 g/L, preferably of from 12 g/L to 13 g/L, particularly preferably of from 12.1 g/L to 12.7 g/L.

Preferably in the method according to the present invention, the concentration of potassium acetate in the growth medium provided in step (i) is 12.1 g/L.

Preferably in the method according to the present invention, the concentration of potassium acetate in the growth medium provided in step (i) is 12.7 g/L.

Preferably in the method according to the present invention, the concentration ratio of glucose to ammonium sulfate in the growth medium provided in step (i) is in the range of from 1 to 6, preferably in the range of from 1.5 to 5.5, preferably in the range of from 2 to 5.5, preferably in the range of from 2.2 to 5.5, preferably in the range of from 2.5 to 5.5, preferably in the range of from 3 to 5.5, preferably in the range of from 3.5 to 5.5, preferably in the range of from 4 to 5.5, particularly preferably in the range of from 5.2 to 5.3.

Preferably in the method according to the present invention, the concentration ratio of glucose to ammonium sulfate in the growth medium provided in step (i) is in the range of from 1 to 6, preferably in the range of from 1.5 to 5.5, preferably in the range of from 2 to 5.5, preferably in the range of from 2.2 to 5.5, preferably in the range of from 2.5 to 5.5, preferably in the range of from 3 to 5.5, preferably in the range of from 3.5 to 5.5, preferably in the range of from 4 to 5.5, particularly preferably in the range of from 5.2 to 5.3, wherein the concentration ratio of glucose to ammonium sulfate is calculated based on mass (or weight) per volume of glucose and ammonium sulfate.

Preferably in the method according to the present invention, the concentration ratio of glucose to ammonium sulfate in the growth medium provided in step (i) is in the range of from 2 to 6, preferably in the range of from 2 to 5.5, preferably in the range of from 2 to 5, preferably in the range of from 2 to 4.5, preferably in the range of from 2 to 4, preferably in the range of from 3 to 3.5, preferably in the range of from 2 to 3, preferably in the range of from 2 to 2.5, particularly preferably in the range of from 2.2 to 2.3.

Preferably in the method according to the present invention, the concentration ratio of glucose to ammonium sulfate in the growth medium provided in step (i) is in the range of from 2 to 6, preferably in the range of from 2 to 5.5, preferably in the range of from 2 to 5, preferably in the range of from 2 to 4.5, preferably in the range of from 2 to 4, preferably

in the range of from 3 to 3.5, preferably in the range of from 2 to 3, preferably in the range of from 2 to 2.5, particularly preferably in the range of from 2.2 to 2.3, wherein the concentration ratio of glucose to ammonium sulfate is calculated based on mass (or weight) per volume of glucose and ammonium sulfate.

Preferably in the method according to the present invention, the concentration ratio of glucose to ammonium sulfate in the growth medium provided in step (i) is 2.21, wherein the concentration ratio of glucose to ammonium sulfate is calculated based on mass (or weight) per volume of glucose and ammonium sulfate.

Preferably in the method according to the present invention, the concentration ratio of glucose to ammonium sulfate in the growth medium provided in step (i) is 5.21, wherein the concentration ratio of glucose to ammonium sulfate is calculated based on mass (or weight) per volume of glucose and ammonium sulfate.

Preferably in the method according to the present invention, the concentration of ammonium sulfate in the growth medium provided in step (i) is in the range of from 7 g/L to 21 g/L, preferably of from 7.5 g/L to 20.5 g/L, preferably of from 8 g/L to 20 g/L, preferably of from 9 g/L to 19 g/L, preferably of from 9.5 g/L to 19.5 g/L, particularly preferably of from 9.6 g/L to 19.3 g/L.

Preferably in the method according to the present invention, the concentration of ammonium sulfate in the growth medium provided in step (i) is 9.6 g/L.

Preferably in the method according to the present invention, the concentration of ammonium sulfate in the growth medium provided in step (i) is 19.3 g/L.

Preferably in the method according to the present invention, the concentration ratio of glucose to urea in the growth medium provided in step (i) is in the range of from 5 to 13, preferably in the range of from 6 to 12.5, preferably in the range of from 6.5 to 12.2, preferably in the range of from 6.5 to 12, preferably in the range of from 6.5 to 10, preferably in the range of from 6.5 to 9, preferably in the range of from 6.5 to 8, preferably in the range of from 6.5 to 7.5, preferably in the range of from 6.5 to 7, particularly preferably in the range of from 6.7 to 6.8.

Preferably in the method according to the present invention, the concentration ratio of glucose to urea in the growth medium provided in step (i) is in the range of from 5 to 13, preferably in the range of from 6 to 12.5, preferably in the range of from 6.5 to 12.2, preferably in the range of from 6.5 to 12, preferably in the range of from 6.5 to 10, preferably in the range of from 6.5 to 9, preferably in the range of from 6.5 to 8, preferably in the range of from 6.5 to 7.5, preferably in the range of from 6.5 to 7, particularly preferably in the range of from 6.7 to 6.8, wherein the concentration ratio of glucose to urea is calculated based on mass (or weight) per volume of glucose and urea.

Preferably in the method according to the present invention, the concentration ratio of glucose to urea in the growth medium provided in step (i) is in the range of from 5 to 13, preferably in the range of from 6 to 12.5, preferably in the range of from 6.5 to 12.2, preferably in the range of from 7 to 12.2, preferably in the range of from 7.5 to 12.2, preferably in the range of from 8 to 12.2, preferably in the range of from 8.5 to 12.2, preferably in the range of from 9 to 12.2, preferably in the range of from 9.5 to 12.2, preferably in the range of from 10 to 12.2, preferably in the range of from 10.5 to 12.2, preferably in the range of from 11 to 12.2, preferably in the range of from 11.5 to 12.2 particularly preferably in the range of from 12.1 to 12.2.

Preferably in the method according to the present invention, the concentration ratio of glucose to urea in the growth medium provided in step (i) is in the range of from 5 to 13, preferably in the range of from 6 to 12.5, preferably in the range of from 6.5 to 12.2, preferably in the range of from 7 to 12.2, preferably in the range of from 7.5 to 12.2, preferably in the range of from 8 to 12.2, preferably in the range of from 8.5 to 12.2, preferably in the range of from 9 to 12.2, preferably in the range of from 9.5 to 12.2, preferably in the range of from 10 to 12.2, preferably in the range of from 10.5 to 12.2, preferably in the range of from 11 to 12.2, preferably in the range of from 11.5 to 12.2 particularly preferably in the range of from 12.1 to 12.2, wherein the concentration ratio of glucose to urea is calculated based on mass (or weight) per volume of glucose and urea.

Preferably in the method according to the present invention, the concentration ratio of glucose to urea in the growth medium provided in step (i) is 6.67, wherein the concentration ratio of glucose to urea is calculated based on mass (or weight) per volume of glucose and urea.

Preferably in the method according to the present invention, the concentration ratio of glucose to urea in the growth medium provided in step (i) is 12.17, wherein the concentration ratio of glucose to urea is calculated based on mass (or weight) per volume of glucose and urea.

Preferably in the method according to the present invention, the concentration of urea in the growth medium provided in step (i) is in the range of from 2.5 g/L to 8.5 g/L, preferably of from 3 g/L to 8 g/L, preferably of from 3.3 g/L to 7.7 g/L, preferably of from 3.4 g/L to 7.6 g/L, preferably of from 3.5 g/L to 7.5 g/L, particularly preferably of from 3.5 g/L to 7.4 g/L.

Preferably in the method according to the present invention, the concentration of urea in the growth medium provided in step (i) is 3.5 g/L.

Preferably in the method according to the present invention, the concentration of urea in the growth medium provided in step (i) is 7.4 g/L.

Preferably in the method according to the present invention, the concentration ratio of glucose to phosphate in the growth medium provided in step (i) is in the range of from 10 to 28, preferably in the range of from 11 to 27, preferably in the

range of from 11 to 25, preferably in the range of from 11 to 20, preferably in the range of from 11 to 18, preferably in the range of from 11 to 16, preferably in the range of from 11 to 14, preferably in the range of from 11 to 13, preferably in the range of from 11 to 12, particularly preferably in the range of from 11.2 to 11.3.

Preferably in the method according to the present invention, the concentration ratio of glucose to phosphate in the growth medium provided in step (i) is in the range of from 10 to 28, preferably in the range of from 11 to 27, preferably in the range of from 11 to 25, preferably in the range of from 11 to 20, preferably in the range of from 11 to 18, preferably in the range of from 11 to 16, preferably in the range of from 11 to 14, preferably in the range of from 11 to 13, preferably in the range of from 11 to 12, particularly preferably in the range of from 11.2 to 11.3, wherein the concentration ratio of glucose to phosphate is calculated based on mass (or weight) per volume of glucose and phosphate.

Preferably in the method according to the present invention, the concentration ratio of glucose to phosphate in the growth medium provided in step (i) is in the range of from 10 to 28, preferably in the range of from 11 to 27, preferably in the range of from 13 to 26.5, preferably in the range of from 15 to 26.5, preferably in the range of from 17 to 26.5, preferably in the range of from 19 to 26.5, preferably in the range of from 21 to 26.5, preferably in the range of from 23 to 26.5, preferably in the range of from 25 to 26.5, particularly preferably in the range of from 26.3 to 26.4.

Preferably in the method according to the present invention, the concentration ratio of glucose to phosphate in the growth medium provided in step (i) is in the range of from 10 to 28, preferably in the range of from 11 to 27, preferably in the range of from 13 to 26.5, preferably in the range of from 15 to 26.5, preferably in the range of from 17 to 26.5, preferably in the range of from 19 to 26.5, preferably in the range of from 21 to 26.5, preferably in the range of from 23 to 26.5, preferably in the range of from 25 to 26.5, particularly preferably in the range of from 26.3 to 26.4, wherein the concentration ratio of glucose to phosphate is calculated based on mass (or weight) per volume of glucose and phosphate.

Preferably in the method according to the present invention, the concentration ratio of glucose to phosphate in the growth medium provided in step (i) is 11.21, wherein the concentration ratio of glucose to urea is calculated based on mass (or weight) per volume of glucose and phosphate.

Preferably in the method according to the present invention, the concentration ratio of glucose to phosphate in the growth medium provided in step (i) is 26.32, wherein the concentration ratio of glucose to urea is calculated based on mass (or weight) per volume of glucose and phosphate.

Preferably in the method according to the present invention, the concentration of phosphate in the growth medium provided in step (i) is in the range of from 1.5 g/L to 4.5 g/L, preferably of from 1.7 g/L to 4 g/L, preferably of from 1.8 g/L to 3.9 g/L, preferably of from 1.9 g/L to 3.9 g/L, particularly preferably of from 1.9 g/L to 3.8 g/L.

Preferably in the method according to the present invention, the concentration of phosphate in the growth medium provided in step (i) is 1.9 g/L.

Preferably in the method according to the present invention, the concentration of phosphate in the growth medium provided in step (i) is 3.8 g/L.

Preferably in the method according to the present invention, the concentration ratio of potassium acetate to glucose in the growth medium provided in step (i) is in the range of from 0.2 to 0.4, preferably in the range of from 0.2 to 0.3, preferably in the range of from 0.25 to 0.3, particularly preferably in the range of from 0.25 to 0.28.

Preferably in the method according to the present invention, the concentration ratio of potassium acetate to glucose in the growth medium provided in step (i) is in the range of from 0.2 to 0.4, preferably in the range of from 0.2 to 0.3, preferably in the range of from 0.25 to 0.3, particularly preferably in the range of from 0.25 to 0.28, wherein the concentration ratio of potassium acetate to glucose is calculated based on mass (or weight) per volume of potassium acetate and glucose.

Preferably in the method according to the present invention, the concentration ratio of potassium acetate to glucose in the growth medium provided in step (i) is 0.25, wherein the concentration ratio of potassium acetate to glucose is calculated based on mass (or weight) per volume of potassium acetate and glucose.

Preferably in the method according to the present invention, the concentration ratio of potassium acetate to glucose in the growth medium provided in step (i) is 0.28, wherein the concentration ratio of potassium acetate to glucose is calculated based on mass (or weight) per volume of potassium acetate and glucose.

Preferably in the method according to the present invention, the concentration ratio of potassium acetate to ammonium sulfate in the growth medium provided in step (i) is in the range of from 0.5 to 1.5, preferably in the range of from 0.6 to 1.4, preferably in the range of from 0.6 to 1.35, particularly preferably in the range of from 0.63 to 1.32.

Preferably in the method according to the present invention, the concentration ratio of potassium acetate to ammonium sulfate in the growth medium provided in step (i) is in the range of from 0.5 to 1.5, preferably in the range of from 0.6 to 1.4, preferably in the range of from 0.6 to 1.35, particularly preferably in the range of from 0.63 to 1.32, wherein the concentration ratio of potassium acetate to glucose is calculated based on mass (or weight) per volume of potassium acetate and ammonium sulfate.

Preferably in the method according to the present invention, the concentration ratio of potassium acetate to ammonium sulfate in the growth medium provided in step (i) is 0.63, wherein the concentration ratio of potassium acetate to ammonium sulfate is calculated based on mass (or weight) per volume of potassium acetate and glucose.

Preferably in the method according to the present invention, the concentration ratio of potassium acetate to ammonium sulfate in the growth medium provided in step (i) is 1.32, wherein the concentration ratio of potassium acetate to ammonium sulfate is calculated based on mass (or weight) per volume of potassium acetate and glucose.

Preferably in the method according to the present invention, the concentration ratio of potassium acetate to urea in the growth medium provided in step (i) is in the range of from 1.5 to 4, preferably in the range of from 1.6 to 3.5, preferably in the range of from 1.7 to 3.5, particularly preferably in the range of from 1.72 to 3.46.

Preferably in the method according to the present invention, the concentration ratio of potassium acetate to urea in the growth medium provided in step (i) is in the range of from 1.5 to 4, preferably in the range of from 1.6 to 3.5, preferably in the range of from 1.7 to 3.5, particularly preferably in the range of from 1.72 to 3.46, wherein the concentration ratio of potassium acetate to glucose is calculated based on mass (or weight) per volume of potassium acetate and urea.

Preferably in the method according to the present invention, the concentration ratio of potassium acetate to urea in the growth medium provided in step (i) is 1.72, wherein the concentration ratio of potassium acetate to ammonium sulfate is calculated based on mass (or weight) per volume of potassium acetate and urea.

Preferably in the method according to the present invention, the concentration ratio of potassium acetate to urea in the growth medium provided in step (i) is 3.46, wherein the concentration ratio of potassium acetate to ammonium sulfate is calculated based on mass (or weight) per volume of potassium acetate and urea.

Preferably in the method according to the present invention, the concentration ratio of potassium acetate to phosphate in the growth medium provided in step (i) is in the range of from 3 to 8, preferably in the range of from 3.1 to 7, preferably in the range of from 3.15 to 6.7, particularly preferably in the range of from 3.18 to 6.68.

Preferably in the method according to the present invention, the concentration ratio of potassium acetate to phosphate in the growth medium provided in step (i) is in the range of from 3 to 8, preferably in the range of from 3.1 to 7, preferably in the range of from 3.15 to 6.7, particularly preferably in the range of from 3.18 to 6.68, wherein the concentration ratio of potassium acetate to glucose is calculated based on mass (or weight) per volume of potassium acetate and phosphate.

Preferably in the method according to the present invention, the concentration ratio of potassium acetate to phosphate in the growth medium provided in step (i) is 3.18, wherein the concentration ratio of potassium acetate to ammonium sulfate is calculated based on mass (or weight) per volume of potassium acetate and urea.

Preferably in the method according to the present invention, the concentration ratio of potassium acetate to phosphate in the growth medium provided in step (i) is 6.68, wherein the concentration ratio of potassium acetate to ammonium sulfate is calculated based on mass (or weight) per volume of potassium acetate and phosphate.

Preferably in the method according to the present invention, the concentration ratio of ammonium sulfate to potassium acetate in the growth medium provided in step (i) is in the range of from 0.5 to 1.8, preferably in the range of from 0.6 to 1.7, preferably in the range of from 0.7 to 1.6, particularly preferably in the range of from 0.76 to 1.6.

Preferably in the method according to the present invention, the concentration ratio of ammonium sulfate to potassium acetate in the growth medium provided in step (i) is in the range of from 0.5 to 1.8, preferably in the range of from 0.6 to 1.7, preferably in the range of from 0.7 to 1.6, particularly preferably in the range of from 0.76 to 1.6, wherein the concentration ratio of potassium acetate to glucose is calculated based on mass (or weight) per volume of ammonium sulfate and potassium acetate.

Preferably in the method according to the present invention, the concentration ratio of ammonium sulfate to potassium acetate in the growth medium provided in step (i) is 0.76, wherein the concentration ratio of potassium acetate to ammonium sulfate is calculated based on mass (or weight) per volume of potassium acetate and urea.

Preferably in the method according to the present invention, the concentration ratio of ammonium sulfate to potassium acetate in the growth medium provided in step (i) is 1.6, wherein the concentration ratio of potassium acetate to ammonium sulfate is calculated based on mass (or weight) per volume of ammonium sulfate and potassium acetate.

Preferably in the method according to the present invention, the concentration ratio of ammonium sulfate to glucose in the growth medium provided in step (i) is in the range of from 0.15 to 0.55, preferably in the range of from 0.17 to 0.5, preferably in the range of from 0.18 to 0.46, particularly preferably in the range of from 0.19 to 0.45.

Preferably in the method according to the present invention, the concentration ratio of ammonium sulfate to glucose in the growth medium provided in step (i) is in the range of from 0.15 to 0.55, preferably in the range of from 0.17 to 0.5, preferably in the range of from 0.18 to 0.46, particularly preferably in the range of from 0.19 to 0.45, wherein the concentration ratio of potassium acetate to glucose is calculated based on mass (or weight) per volume of ammonium sulfate and glucose.

Preferably in the method according to the present invention, the concentration ratio of ammonium sulfate to glucose in the growth medium provided in step (i) is 0.19, wherein the concentration ratio of potassium acetate to ammonium sulfate is calculated based on mass (or weight) per volume of potassium acetate and urea.

Preferably in the method according to the present invention, the concentration ratio of ammonium sulfate to glucose in the growth medium provided in step (i) is 0.45, wherein the concentration ratio of potassium acetate to ammonium sulfate is calculated based on mass (or weight) per volume of ammonium sulfate and glucose.

Preferably in the method according to the present invention, the concentration ratio of ammonium sulfate to urea in the growth medium provided in step (i) is in the range of from 1.1 to 6.5, preferably in the range of from 1.2 to 6, preferably in the range of from 1.3 to 5.6, particularly preferably in the range of from 1.3 to 5.51.

Preferably in the method according to the present invention, the concentration ratio of ammonium sulfate to urea in the growth medium provided in step (i) is in the range of from 1.1 to 6.5, preferably in the range of from 1.2 to 6, preferably in the range of from 1.3 to 5.6, particularly preferably in the range of from 1.3 to 5.51, wherein the concentration ratio of potassium acetate to glucose is calculated based on mass (or weight) per volume of ammonium sulfate and urea.

Preferably in the method according to the present invention, the concentration ratio of ammonium sulfate to urea in the growth medium provided in step (i) is 1.3, wherein the concentration ratio of potassium acetate to ammonium sulfate is calculated based on mass (or weight) per volume of potassium acetate and urea.

Preferably in the method according to the present invention, the concentration ratio of ammonium sulfate to urea in the growth medium provided in step (i) is 5.51, wherein the concentration ratio of potassium acetate to ammonium sulfate is calculated based on mass (or weight) per volume of ammonium sulfate and urea.

Preferably in the method according to the present invention, the concentration ratio of ammonium sulfate to phosphate in the growth medium provided in step (i) is in the range of from 5 to 5.1, particularly preferably in the range of from 5.05 to 5.08.

Preferably in the method according to the present invention, the concentration ratio of ammonium sulfate to phosphate in the growth medium provided in step (i) is in the range of from 5 to 5.1, particularly preferably in the range of from 5.05 to 5.08, wherein the concentration ratio of potassium acetate to glucose is calculated based on mass (or weight) per volume of ammonium sulfate and phosphate.

Preferably in the method according to the present invention, the concentration ratio of ammonium sulfate to phosphate in the growth medium provided in step (i) is 5.05, wherein the concentration ratio of potassium acetate to ammonium sulfate is calculated based on mass (or weight) per volume of potassium acetate and urea.

Preferably in the method according to the present invention, the concentration ratio of ammonium sulfate to phosphate in the growth medium provided in step (i) is 5.08, wherein the concentration ratio of potassium acetate to ammonium sulfate is calculated based on mass (or weight) per volume of ammonium sulfate and phosphate.

Preferably in the method according to the present invention, the concentration ratio of urea to potassium acetate in the growth medium provided in step (i) is in the range of from 0.25 to 0.65, preferably in the range of from 0.27 to 0.6, preferably in the range of from 0.28 to 0.59, particularly preferably in the range of from 0.29 to 0.58.

Preferably in the method according to the present invention, the concentration ratio of urea to potassium acetate in the growth medium provided in step (i) is in the range of from 0.25 to 0.65, preferably in the range of from 0.27 to 0.6, preferably in the range of from 0.28 to 0.59, particularly preferably in the range of from 0.29 to 0.58, wherein the concentration ratio of potassium acetate to glucose is calculated based on mass (or weight) per volume of urea and potassium acetate.

Preferably in the method according to the present invention, the concentration ratio of urea to potassium acetate in the growth medium provided in step (i) is 0.29, wherein the concentration ratio of potassium acetate to ammonium sulfate is calculated based on mass (or weight) per volume of urea and potassium acetate.

Preferably in the method according to the present invention, the concentration ratio of urea to potassium acetate in the growth medium provided in step (i) is 0.58, wherein the concentration ratio of potassium acetate to ammonium sulfate is calculated based on mass (or weight) per volume of urea and potassium acetate.

Preferably in the method according to the present invention, the concentration ratio of urea to glucose in the growth medium provided in step (i) is in the range of from 0.05 to 0.2, preferably in the range of from 0.06 to 0.18, preferably in the range of from 0.07 to 0.16, particularly preferably in the range of from 0.08 to 0.15.

Preferably in the method according to the present invention, the concentration ratio of urea to glucose in the growth medium provided in step (i) is in the range of from 0.05 to 0.2, preferably in the range of from 0.06 to 0.18, preferably in the range of from 0.07 to 0.16, particularly preferably in the range of from 0.08 to 0.15, wherein the concentration ratio of potassium acetate to glucose is calculated based on mass (or weight) per volume of urea and glucose.

Preferably in the method according to the present invention, the concentration ratio of urea to glucose in the growth medium provided in step (i) is 0.08, wherein the concentration ratio of potassium acetate to ammonium sulfate is calculated based on mass (or weight) per volume of potassium acetate and urea.

Preferably in the method according to the present invention, the concentration ratio of urea to glucose in the growth medium provided in step (i) is 0.15, wherein the concentration ratio of urea to ammonium sulfate is calculated based on mass (or weight) per volume of urea and glucose.

Preferably in the method according to the present invention, the concentration ratio of urea to ammonium sulfate in the growth medium provided in step (i) is in the range of from 0.15 to 0.9, preferably in the range of from 0.16 to 0.85, preferably in the range of from 0.17 to 0.8, particularly preferably in the range of from 0.18 to 0.77.

Preferably in the method according to the present invention, the concentration ratio of urea to ammonium sulfate in the growth medium provided in step (i) is in the range of from 0.15 to 0.9, preferably in the range of from 0.16 to 0.85,

preferably in the range of from 0.17 to 0.8, particularly preferably in the range of from 0.18 to 0.77, wherein the concentration ratio of urea to ammonium sulfate is calculated based on mass (or weight) per volume of urea and ammonium sulfate.

Preferably in the method according to the present invention, the concentration ratio of urea to ammonium sulfate in the growth medium provided in step (i) is 0.18, wherein the concentration ratio of urea to ammonium sulfate is calculated based on mass (or weight) per volume of urea and ammonium sulfate.

Preferably in the method according to the present invention, the concentration ratio of urea to glucose in the growth medium provided in step (i) is 0.77, wherein the concentration ratio of potassium acetate to ammonium sulfate is calculated based on mass (or weight) per volume of urea and ammonium sulfate.

Preferably in the method according to the present invention, the concentration ratio of urea to phosphate in the growth medium provided in step (i) is in the range of from 0.8 to 4.5, preferably in the range of from 0.85 to 4, preferably in the range of from 0.9 to 4, particularly preferably in the range of from 0.92 to 3.89.

Preferably in the method according to the present invention, the concentration ratio of urea to phosphate in the growth medium provided in step (i) is in the range of from 0.8 to 4.5, preferably in the range of from 0.85 to 4, preferably in the range of from 0.9 to 4, particularly preferably in the range of from 0.92 to 3.89, wherein the concentration ratio of urea to phosphate is calculated based on mass (or weight) per volume of urea and phosphate.

Preferably in the method according to the present invention, the concentration ratio of urea to phosphate in the growth medium provided in step (i) is 0.92, wherein the concentration ratio of urea to phosphate is calculated based on mass (or weight) per volume of urea and phosphate.

Preferably in the method according to the present invention, the concentration ratio of urea to phosphate in the growth medium provided in step (i) is 3.89, wherein the concentration ratio of urea to phosphate is calculated based on mass (or weight) per volume of urea and phosphate.

Preferably in the method according to the present invention, the concentration ratio of phosphate to potassium acetate in the growth medium provided in step (i) is in the range of from 0.1 to 0.4, preferably in the range of from 0.12 to 0.35, preferably in the range of from 0.13 to 0.32, particularly preferably in the range of from 0.15 to 0.31.

Preferably in the method according to the present invention, the concentration ratio of phosphate to potassium acetate in the growth medium provided in step (i) is in the range of from 0.1 to 0.4, preferably in the range of from 0.12 to 0.35, preferably in the range of from 0.13 to 0.32, particularly preferably in the range of from 0.15 to 0.31, wherein the concentration ratio of phosphate to potassium acetate is calculated based on mass (or weight) per volume of phosphate and potassium acetate.

Preferably in the method according to the present invention, the concentration ratio of phosphate to potassium acetate in the growth medium provided in step (i) is 0.15, wherein the concentration ratio of phosphate to potassium acetate is calculated based on mass (or weight) per volume of phosphate and potassium acetate.

Preferably in the method according to the present invention, the concentration ratio of phosphate to potassium acetate in the growth medium provided in step (i) is 0.31, wherein the concentration ratio of phosphate to potassium acetate is calculated based on mass (or weight) per volume of phosphate and potassium acetate.

Preferably in the method according to the present invention, the concentration ratio of phosphate to glucose in the growth medium provided in step (i) is in the range of from 0.02 to 0.11, preferably in the range of from 0.03 to 0.1, particularly preferably in the range of from 0.04 to 0.09.

Preferably in the method according to the present invention, the concentration ratio of phosphate to glucose in the growth medium provided in step (i) is in the range of from 0.02 to 0.11, preferably in the range of from 0.03 to 0.1, particularly preferably in the range of from 0.04 to 0.09, wherein the concentration ratio of phosphate to glucose is calculated based on mass (or weight) per volume of phosphate and glucose.

Preferably in the method according to the present invention, the concentration ratio of phosphate to glucose in the growth medium provided in step (i) is 0.04, wherein the concentration ratio of phosphate to glucose is calculated based on mass (or weight) per volume of phosphate and glucose.

Preferably in the method according to the present invention, the concentration ratio of phosphate to glucose in the growth medium provided in step (i) is 0.09, wherein the concentration ratio of phosphate to glucose is calculated based on mass (or weight) per volume of phosphate and glucose.

Preferably in the method according to the present invention, the concentration ratio of phosphate to ammonium sulfate in the growth medium provided in step (i) is in the range of from 0.1 to 0.3, preferably in the range of from 0.15 to 0.25, particularly preferably 0.2.

Preferably in the method according to the present invention, the concentration ratio of phosphate to ammonium sulfate in the growth medium provided in step (i) is in the range of from 0.1 to 0.3, preferably in the range of from 0.15 to 0.25, particularly preferably 0.2, wherein the concentration ratio of phosphate to ammonium sulfate is calculated based on mass (or weight) per volume of phosphate and ammonium sulfate.

Preferably in the method according to the present invention, the concentration ratio of phosphate to ammonium sulfate in the growth medium provided in step (i) is 0.2.

Preferably in the method according to the present invention, the concentration ratio of phosphate to urea in the growth medium provided in step (i) is in the range of from 0.2 to 1.2, preferably in the range of from 0.24 to 1.1, particularly preferably in the range of from 0.26 to 1.09.

Preferably in the method according to the present invention, the concentration ratio of phosphate to urea in the growth medium provided in step (i) is in the range of from 0.2 to 1.2, preferably in the range of from 0.24 to 1.1, particularly preferably in the range of from 0.26 to 1.09, wherein the concentration ratio of phosphate to urea is calculated based on mass (or weight) per volume of phosphate and urea.

Preferably in the method according to the present invention, the concentration ratio of phosphate to urea in the growth medium provided in step (i) is 0.26, wherein the concentration ratio of phosphate to glucose is calculated based on mass (or weight) per volume of phosphate and glucose.

Preferably in the method according to the present invention, the concentration ratio of phosphate to urea in the growth medium provided in step (i) is 1.09, wherein the concentration ratio of phosphate to urea is calculated based on mass (or weight) per volume of phosphate and urea.

Preferably in the method according to the present invention, the concentration ratio of glucose to potassium acetate in the growth medium provided in step (i) is 3.94, wherein the concentration of glucose in the growth medium provided in step (i) is 50 g/L and the concentration of potassium acetate in the growth medium provided in step (i) is 12.7 g/L,
and/or

> the concentration ratio of glucose to ammonium sulfate in the growth medium provided in step (i) is 5.21, wherein the concentration of glucose in the growth medium provided in step (i) is 50 g/L and the concentration of ammonium sulfate in the growth medium provided in step (i) is 9.6 g/L,
> and/or

> the concentration ratio of glucose to urea in the growth medium provided in step (i) is 6.76, wherein the concentration of glucose in the growth medium provided in step (i) is 50 g/L and the concentration of urea in the growth medium provided in step (i) is 7.4 g/L,
> and/or

> the concentration ratio of glucose to phosphate in the growth medium provided in step (i) is 6.76, wherein the concentration of glucose in the growth medium provided in step (i) is 50 g/L and the concentration of phosphate in the growth medium provided in step (i) is 1.9 g/L.

Preferably in the method according to the present invention, the concentration ratio of glucose to potassium acetate in the growth medium provided in step (i) is 3.52, wherein the concentration of glucose in the growth medium provided in step (i) is 42.6 g/L and the concentration of potassium acetate in the growth medium provided in step (i) is 12.1 g/L,
and/or

> the concentration ratio of glucose to ammonium sulfate in the growth medium provided in step (i) is 2.21, wherein the concentration of glucose in the growth medium provided in step (i) is 42.6 g/L and the concentration of ammonium sulfate in the growth medium provided in step (i) is 19.3 g/L,
> and/or

> the concentration ratio of glucose to urea in the growth medium provided in step (i) is 12.17, wherein the concentration of glucose in the growth medium provided in step (i) is 42.6 g/L and the concentration of urea in the growth medium provided in step (i) is 3.5 g/L,
> and/or

> the concentration ratio of glucose to phosphate in the growth medium provided in step (i) is 11.21, wherein the concentration of glucose in the growth medium provided in step (i) is 42.6 g/L and the concentration of phosphate in the growth medium provided in step (i) is 3.8 g/L.

Surprisingly, it was found that selecting at least one, preferably at least two, preferably at least three, particularly preferably all four of the concentration ratios of glucose to potassium acetate, glucose to ammonium sulfate, glucose to urea, and glucose to phosphate according to the present invention as described herein led to an increase in biomass formation of the one or more producer organism(s) used for the production of one or more species of carotenoids.

Surprisingly, it was found that selecting at least one, preferably at least two, preferably at least three, particularly

preferably all four of the concentration ratios of glucose to potassium acetate, glucose to ammonium sulfate, glucose to urea, and glucose to phosphate according to the present invention as described herein led to an increase in the biomass formation rate, i.e. growth rate, of the one or more producer organism(s) used for the production of one or more species of carotenoids.

Surprisingly, it was also found that selecting at least one, preferably at least two, preferably at least three, particularly preferably all four of the concentration ratios of glucose to potassium acetate, glucose to ammonium sulfate, glucose to urea, and glucose to phosphate according to the present invention as described herein led to an increase in the overall titer of produced carotenoids.

Surprisingly, it was also found that selecting at least one, preferably at least two, preferably at least three, particularly preferably all four of the concentration ratios of glucose to potassium acetate, glucose to ammonium sulfate, glucose to urea, and glucose to phosphate according to the present invention as described herein led to an increase in the titer of produced astaxanthin.

Thus, selecting at least one, preferably at least two, preferably at least three, particularly preferably all four of the concentration ratios of glucose to potassium acetate, glucose to ammonium sulfate, glucose to urea, and glucose to phosphate according to the present invention as described herein allows for producing one or more species of carotenoids, particularly astaxanthin either alone or in combination with one or more other species of carotenoids, in a particularly cost-efficient, time-efficient, resource-efficient, and sustainable manner.

Surprisingly, it was also found that selecting the concentrations of at least one, preferably at least two, preferably at least three, preferably at least four, particularly preferably all five, of the compounds selected from the group consisting of glucose, potassium acetate, ammonium sulfate, urea, and phosphate according to present invention as described herein also lead to an increase in biomass formation of the one or more producer organism(s) used for the production of one or more species of carotenoids.

Surprisingly, it was also found that selecting the concentrations of at least one, preferably at least two, preferably at least three, preferably at least four, particularly preferably all five of the compounds selected from the group consisting of glucose, potassium acetate, ammonium sulfate, urea, and phosphate according to present invention as described herein also lead to an increase in the biomass formation rate, i.e. growth rate, of the one or more producer organism(s) used for the production of one or more species of carotenoids.

Surprisingly, it was also found that selecting the concentrations of at least one, preferably at least two, preferably at least three, preferably at least four, particularly preferably all five of the compounds selected from the group consisting of glucose, potassium acetate, ammonium sulfate, urea, and phosphate according to the present invention as described herein led to an increase in the overall titer of produced carotenoids.

Surprisingly, it was also found that selecting the concentrations of at least one, preferably at least two, preferably at least three, preferably at least four, particularly preferably all five of the compounds selected from the group consisting of glucose, potassium acetate, ammonium sulfate, urea, and phosphate according to the present invention as described herein led to an increase in the titer of produced astaxanthin.

Thus, selecting the concentrations of at least one, preferably at least two, preferably at least three, preferably at least four, particularly preferably all five of the compounds selected from the group consisting of glucose, potassium acetate, ammonium sulfate, urea, and phosphate according to present invention as described herein allows for producing one or more species of carotenoids, particularly astaxanthin either alone or in combination with one or more other species of carotenoids, in a particularly cost-efficient, time-efficient, resource-efficient, and sustainable manner.

Preferably in the method according to the present invention, the cultivation conditions allowing production of one or more species of carotenoids may be selected from the group consisting of oxic conditions, anoxic conditions, microoxic conditions, wherein one or more different electron acceptor(s) may be provided, which may preferably be independently selected from the non-limiting group consisting of oxygen, sulfate, nitrate, fumarate, iron(III), manganese(IV), partially oxidized metabolic intermediates for one or more different fermentation processes.

Preferably in the method according to the present invention, cultivating one or more producer organism(s) may be performed in bioreactors, preferably automated bioreactors, as a batch cultivation and/or as a continuous cultivation.

Preferably in the method according to the present invention, cultivating one or more producer organism(s) may be performed as a batch cultivation in Erlenmeyer flasks and/or other cultivation vessels, preferably wherein the Erlenmeyer flasks and/or other cultivation vessels are incubated on a rotary shaker or stationarily.

Preferably in the method according to the present invention, cultivating one or more producer organism(s) may be performed as a batch cultivation on solid growth medium, preferably solid plate cultures, preferably agar plates.

Preferably in the method according to the present invention, cultivating one or more producer organism(s) may include monitoring cell growth via one or more methods selected from the non-limiting group consisting of measuring optical density of the culture, measuring the amount(s) of one or more suitable metabolites produced by the one or more producer organism(s), measuring the amount(s) of one or more carbon source(s) metabolized (i.e. oxidized) by the one or more producer organism(s), measuring the enzyme activity of one or more enzyme(s) produced and/or secreted by the one or more producer organism(s), measuring the amount(s) of one or more suitable pigments produced and/or excreted

by the one or more producer organism(s).

Preferably in the method according to the present invention, extracting the one or more species of carotenoids may be achieved by means of centrifugation, preferably in combination with disrupting the cells of the one or more producer organism(s) and/or selectively dissolving a fraction of the cell suspension comprising the one or more species of carotenoids, preferably only one or more species of carotenoids, and/or purifying the one or more species of carotenoids by means of chromatography, preferably high performance liquid chromatography (HPLC), preferably reversed phase HPLC (rpHPLC).

Preferably in the method according to the present invention, the method comprises the additional step of quantifying the one or more species of carotenoids. Preferably, quantifying the one or more species of carotenoids may be performed via high performance liquid chromatography (HPLC), preferably reversed phase HPLC (rpHPLC), preferably reversed phase HPLC (rpHPLC) employing a C18 separation column.

Preferably in the method according to the present invention, cultivating said one or more producer organism(s) is carried out at a temperature in the range of from 5°C to 50°C, preferably of from 10°C to 45°C, preferably of from 15°C to 40°C, preferably of from 18°C to 35°C, preferably of form 20°C to 32 °C, preferably of from 22°C to 32°C, preferably at 30°C.

Preferably in the method according to the present invention, the growth medium provided in step (i) has a pH in the range of from 2 to 10, preferably of from 4 to 9, preferably of form 5 to 8.5, preferably of from 6 to 9, preferably of from 7 to 8.

In a preferred embodiment of the method according to the present invention, the, one, two, three or more or all species of carotenoids is/are selected from the group consisting of astaxanthin, lycopene, β-carotene, echinenone, canthaxanthin, cryptoxanthin, hydroxyechinenone, zeaxanthin, adonirubin, and decaprenoxanthin, preferably astaxanthin, decaprenoxanthin, β-carotene, and canthaxanthin, particularly preferably astaxanthin.

Preferably in the method according to the present invention, the, one, two, three or more or all species of carotenoids is/are selected from the group consisting of astaxanthin, lycopene, β-carotene, echinenone, canthaxanthin, cryptoxanthin, hydroxyechinenone, zeaxanthin, adonirubin, and decaprenoxanthin, lutein, β-cryptoxanthin, 3'-hydroxyechinenone, adonixanthin, preferably astaxanthin, decaprenoxanthin, β-carotene, and canthaxanthin, particularly preferably astaxanthin.

Preferably in the method according to the present invention, the one or more species of carotenoids is/are selected from the group consisting of astaxanthin, lycopene, β-carotene, echinenone, canthaxanthin, cryptoxanthin, hydroxyechinenone, zeaxanthin, adonirubin, and decaprenoxanthin, lutein, β-cryptoxanthin, 3'-hydroxyechinenone, adonixanthin, preferably astaxanthin, decaprenoxanthin, β-carotene, and canthaxanthin, particularly preferably astaxanthin, and

the concentration ratio of glucose to potassium acetate in the growth medium provided in step (i) is 3.94, wherein the concentration of glucose in the growth medium provided in step (i) is 50 g/L and the concentration of potassium acetate in the growth medium provided in step (i) is 12.7 g/L,
and/or

the concentration ratio of glucose to ammonium sulfate in the growth medium provided in step (i) is 5.21, wherein the concentration of glucose in the growth medium provided in step (i) is 50 g/L and the concentration of ammonium sulfate in the growth medium provided in step (i) is 9.6 g/L,
and/or

the concentration ratio of glucose to urea in the growth medium provided in step (i) is 6.76, wherein the concentration of glucose in the growth medium provided in step (i) is 50 g/L and the concentration of urea in the growth medium provided in step (i) is 7.4 g/L,
and/or

the concentration ratio of glucose to phosphate in the growth medium provided in step (i) is 6.76, wherein the concentration of glucose in the growth medium provided in step (i) is 50 g/L and the concentration of phosphate in the growth medium provided in step (i) is 1.9 g/L.

Preferably in the method according to the present invention, the one or more species of carotenoids is/are selected from the group consisting of astaxanthin, lycopene, β-carotene, echinenone, canthaxanthin, cryptoxanthin, hydroxyechinenone, zeaxanthin, adonirubin, and decaprenoxanthin, lutein, β-cryptoxanthin, 3'-hydroxyechinenone, adonixanthin, preferably astaxanthin, decaprenoxanthin, β-carotene, and canthaxanthin, particularly preferably astaxanthin, and

the concentration ratio of glucose to potassium acetate in the growth medium provided in step (i) is 3.52, wherein the

concentration of glucose in the growth medium provided in step (i) is 42.6 g/L and the concentration of potassium acetate in the growth medium provided in step (i) is 12.1 g/L,
and/or

the concentration ratio of glucose to ammonium sulfate in the growth medium provided in step (i) is 2.21, wherein the concentration of glucose in the growth medium provided in step (i) is 42.6 g/L and the concentration of ammonium sulfate in the growth medium provided in step (i) is 19.3 g/L,
and/or

the concentration ratio of glucose to urea in the growth medium provided in step (i) is 12.17, wherein the concentration of glucose in the growth medium provided in step (i) is 42.6 g/L and the concentration of urea in the growth medium provided in step (i) is 3.5 g/L,
and/or

the concentration ratio of glucose to phosphate in the growth medium provided in step (i) is 11.21, wherein the concentration of glucose in the growth medium provided in step (i) is 42.6 g/L and the concentration of phosphate in the growth medium provided in step (i) is 3.8 g/L.

Surprisingly, it was found that selecting at least one, preferably at least two, preferably at least three, particularly preferably all four of the concentration ratios of glucose to potassium acetate, glucose to ammonium sulfate, glucose to urea, and glucose to phosphate according to the present invention as described herein while also selecting the concentrations of at least one, preferably at least two, preferably at least three, preferably at least four, particularly preferably all five of the compounds selected from the group consisting of glucose, potassium acetate, ammonium sulfate, urea, and phosphate according to present invention as described herein, the titer of astaxanthin produced either alone or in combination with one or more other species of carotenoids could be increased.

Surprisingly, it was found that selecting at least one, preferably at least two, preferably at least three, particularly preferably all four of the concentration ratios of glucose to potassium acetate, glucose to ammonium sulfate, glucose to urea, and glucose to phosphate according to the present invention as described herein while also selecting the concentrations of at least one, preferably at least two, preferably at least three, preferably at least four, particularly preferably all five of the compounds selected from the group consisting of glucose, potassium acetate, ammonium sulfate, urea, and phosphate according to present invention as described herein, the titer of astaxanthin produced either alone or in combination with one or more other species of carotenoids could be increased to at least 10 mg/L.

Surprisingly, it was found that the titer of astaxanthin produced either alone or in combination with one or more other species of carotenoids could be particularly drastically increased when using glucose at a concentration of 50 g/L, potassium acetate at a concentration of 12.7 g/L, ammonium sulfate at a concentration of 9.6 g/L, urea at a concentration of 7.4 g/L, and phosphate at a concentration of 1.8 g/L.

Surprisingly, it was found that when using glucose at a concentration of 50 g/L, potassium acetate at a concentration of 12.7 g/L, ammonium sulfate at a concentration of 9.6 g/L, urea at a concentration of 7.4 g/L, and phosphate at a concentration of 1.8 g/L a titer of astaxanthin produced either alone or in combination with one or more other species of carotenoids of at least 12 mg/L could be reached.

Surprisingly, it was also found that the titer of astaxanthin produced either alone or in combination with one or more other species of carotenoids could be particularly drastically increased when using glucose at a concentration of 42.6 g/L, potassium acetate at a concentration of 19.3 g/L, ammonium sulfate at a concentration of 3.5 g/L, urea at a concentration of 3.5 g/L, and phosphate at a concentration of 3.8 g/L.

Surprisingly, it was found that when using glucose at a concentration of 42.6 g/L, potassium acetate at a concentration of 19.3 g/L, ammonium sulfate at a concentration of 3.5 g/L, urea at a concentration of 3.5 g/L, and phosphate at a concentration of 3.8 g/L a titer of astaxanthin produced either alone or in combination with one or more other species of carotenoids of at least 18 mg/L could be reached.

Preferably in the method according to the present invention, the, one, two or three or more or all producer organism(s) added in step (ii) is/are one or more microbial producer organism(s), preferably one or more bacterial producer organism(s), preferably selected from the phylum *Actinomycetota,* preferably selected from the class *Actinomycetia,* preferably selected from the order *Mycobacteriales,* preferably selected from the family *Corynebacteriaceae,* preferably selected from the genus *Corynebacterium,* particularly preferably the species *Corynebacterium glutamicum.*

Preferably in the method according to the present invention, the one or more producer organism(s) added in step (ii) comprise(s), preferably is/are, one or more species and/or genera of yeast, preferably selected from the group consisting of *Phaffia rhodozyma,* Xanthophyllomyces dendrorhous, one or more species of the genus *Rhodosporidium,* one or more species of the genus *Rhodotorula,* one or more species of the genus *Sporobolomyces,* and one or more species of the genus *Sporidiobolus.*

Using yeast, particularly *Phaffia rhodozyma* and/or *Xanthophyllomyces dendrorhous,* as producer organism(s) has the advantage that the risk of contamination is markedly reduced as compared to other producer organism(s), particularly as compared to bacterial producer organisms.

Preferably in the method according to the present invention, the one or more producer organism(s) added in step (ii) comprise(s), preferably is/are, one or more bacterial producer organism(s), preferably selected from the group consisting of *Escherichia coli,* and *Corynebacterium glutamicum.*

Using bacterial producer organism(s) has the advantage that the overall production time and overall production costs can be markedly reduced as compared to other producer organism(s). Moreover, bacterial producer organism(s) allow for high scale-up capacity, easy propagation, and high product yield as compared to other producer organisms.

Preferably in the method according to the present invention, the one or more producer organism(s) added in step (ii) comprise(s), preferably is/are, one or more species of algae, preferably of the division *Chlorophyta,* preferably of the class *Chlorophyceae,* preferably of the order *Chlamydomonadales,* preferably of the family *Haematococcaceae,* preferably of the genus *Haematococcus,* particularly preferably *Haematococcus pluvialis.*

It was found that when selecting the one or more producer organism(s) according to the present invention as described herein, the 3'S 3S enantiomer is the predominating, or even the only, enantiomeric form of all carotenoids produced, particularly of astaxanthin. The enantionmeric purity (enantiopurity) of the produced carotenoids can be determined by methods known to the skilled person, such as gas chromatography and/or nuclear magnetic resonance (NMR) spectroscopy. Preferably, the term "predominating enantiomeric form" describes that at least 30 %, preferably at least 40 %, preferably at least 50 %, preferably at least 60 %, preferably at least 70 %, preferably at least 75 %, preferably at least 80 %, preferably at least 85 %, preferably at least 90 %, preferably at least 95 % of the carotenoid molecules, preferably astaxanthin molecules, are in said enantiomeric form.

It was found that using the bacterial species *Corynebacterium glutamicum* as a producer organism leads to an advantageously high biomass formation and biomass formation rate, i.e. growth rate, of the producer organism.

It was also found that using the bacterial species *Corynebacterium glutamicum* as a producer organism leads to enantiopurity of the 3'S 3S enantiomer of all carotenoids produced (only the 3'S 3S enantiomer was obtained), particularly of astaxanthin. Thus, additional time intense, cost intense, resource intense, and labour intense steps for selectively purifying the 3'S 3S enantiomer of one or more given carotenoids, particularly astaxanthin, can be avoided.

It was found that using the bacterial species *Corynebacterium glutamicum* as a producer organism while also selecting at least one, preferably at least two, preferably at least three, particularly preferably all four of the concentration ratios of glucose to potassium acetate, glucose to ammonium sulfate, glucose to urea, and glucose to phosphate according to the present invention as described herein while also selecting the concentrations of at least one, preferably at least two, preferably at least three, preferably at least four, particularly preferably all five, of the compounds selected from the group consisting of glucose, potassium acetate, ammonium sulfate, urea, and phosphate according to present invention as described herein leads to biomass formation rate, i.e. growth rate, of the producer organism of or beyond 0.3 /h.

Thus, using the bacterial species *Corynebacterium glutamicum* as a producer organism, particularly while also selecting at least one, preferably at least two, preferably at least three, particularly preferably all four of the concentration ratios of glucose to potassium acetate, glucose to ammonium sulfate, glucose to urea, and glucose to phosphate according to the present invention as described herein while also selecting the concentrations of at least one, preferably at least two, preferably at least three, preferably at least four, particularly preferably all five of the compounds selected from the group consisting of glucose, potassium acetate, ammonium sulfate, urea, and phosphate according to present invention as described herein, allows for producing one or more species of carotenoids, particularly astaxanthin either alone or in combination with one or more other species of carotenoids, in a particularly cost-efficient, time-efficient, resource-efficient, and sustainable manner.

Preferably in the method according to the present invention, the, one, two or three or more or all producer organism(s) added in step (ii) comprise(s) an exogeneously expressed lycopene cyclase enzyme CrtY and/or an exogeneously expressed β-carotene ketolase enzyme CrtW and/or an exogenously expressed β-carotene hydroxylase enzyme CrtZ,

wherein the exogenously expressed lycopene cyclase enzyme CrtY has an amino acid sequence according to SEQ ID NO: 1 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, sequence similarity to the amino acid sequence according to SEQ ID No: 1,
and/or

wherein the exogenously expressed β-carotene ketolase enzyme CrtW has an amino acid sequence according to SEQ ID NO: 2 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, sequence similarity to the amino acid sequence according to SEQ ID No: 2,
and/or

wherein the exogenously expressed β-carotene hydroxylase enzyme CrtZ has an amino acid sequence according to SEQ ID NO: 3 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, sequence similarity to the amino acid sequence according to SEQ ID No: 3

Preferably in the method according to the present invention, the, one, two or three or more or all producer organism(s) added in step (ii) comprise(s) an exogenously expressed lycopene cyclase gene *crtY* and/or an exogenously expressed β-carotene ketolase gene *crtW* and/or an exogenously expressed β-carotene hydroxylase gene *crtZ,*

wherein the exogeneously expressed lycopene cyclase gene *crtY* has a nucleotide sequence according to SEQ ID NO: 4 or a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 4,
and/or

wherein the exogeneously expressed β-carotene ketolase gene *crrW* has a nucleotide sequence according to SEQ ID NO: 5 or a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 5,
and/or

wherein the exogenously expressed β-carotene hydroxylase gene *crtZ* has a nucleotide sequence according to SEQ ID NO: 6 or a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 6.

The term "expression" as used herein in the context of genes and/or enzymes refers to the process of utilizing the information of a gene for the synthesis of a functional gene product. Functional gene products can for example be proteins, such as enzymes, or non-coding RNAs, such as transfer-RNAs (tRNA) and small nuclear RNA (snRNA). Thus, the term "expression" as used herein preferably refers to a process comprising one or more of the steps of transcription, RNA splicing (where applicable), translation, and post-translational modification. For example, expression of a protein (such as

an enzyme) from a gene at least comprises the steps of transcription and translation and optionally comprises the steps of RNA splicing (where applicable) and post-translational modification.

Advantageously, extrageneous expression of one or more genes selected from the group consisting of lycopene cyclase *crtY,* β-carotene ketolase *crtW,* and β-carotene hydroxylase *crtZ* as well as extrageneous expression of one or more enzymes selected from the group consisting of lycopene cyclase CrtY, β-carotene ketolase CrtW, and β-carotene hydroxylase CrtZ allows for a fine-tuned optimization of the ratio of enzyme quantities (e.g. lycopene cyclase CrtY to β-carotene ketolase CrtW to β-carotene hydroxylase CrtZ) leading to an optimized and variably adjustable titer of produced carotenoids. For example, this fine-tuned optimization of the ratio of enzyme quantities can be achieved via variation of translation initiation rates (TIR) based on different ribosomal binding sites, spacing lengths, and translation start codons.

Preferably in the method according to the present invention, the genes coding for extrageneously expressed lycopene cyclase enzyme CrtY, β-carotene ketolase enzyme CrtW, and β-carotene hydroxylase enzyme CrtZ are encoded adjacently in a combinatorial gene assembly as part of an expression construct or vector, wherein the specific order of the genes coding for extrageneously expressed lycopene cyclase enzyme CrtY, β-carotene ketolase enzyme CrtW, and β-carotene hydroxylase enzyme CrtZ is variable.

The term "adjacent" as used herein in the context of a combinatorial gene assembly and/or the location of genes in a larger nucleic acid molecule in general refers to two or more genes, wherein the stop codon of one gene and the start codon of another gene are separated by no more than 20 bp.

Preferably in the method according to the present invention, the stop codon of a first gene and the start codon of an adjacently coded gene are separated by a ribosomal binding site and a spacing sequence, wherein said ribosomal binding site has a size of 9 bp and the spacing sequence has a size of 10 bp or less, preferably 8 bp or less, preferably 6 bp or less, preferably 3 bp or less. Preferably, the ribosomal binding site has a sequence according to any one of the sequences selected from the group consisting of SEQ ID NO 7, SEQ ID NO 8, SEQ ID NO 9, and SEQ ID NO 10 and/or the spacing sequence has a sequence according to any one of the sequences selected from the group consisting of SEQ ID NO 11, SEQ ID NO 12, and SEQ ID NO 13.

Preferably in the method according to the present invention, the genes coding for exogenously expressed lycopene cyclase enzyme CrtY, β-carotene ketolase enzyme CrtW, and β-carotene hydroxylase enzyme CrtZ each comprise a start codon, which is either ATG or GTG, preferably ATG.

Advantageously, by separating the genes coding for exogenously expressed lycopene cyclase enzyme CrtY, β-carotene ketolase enzyme CrtW, and β-carotene hydroxylase enzyme CrtZ each by a ribosomal binding site and a spacing sequence according to the present invention as described herein, the transcription initiation rates of each of these exogenously expressed enzymes could be optimized and thus the titer of astaxanthin produced either alone or in combination with one or more other species of carotenoids could be increased.

Preferably in the method according to the present invention, the one or more producer organism(s) added in step (ii) do/does not comprise a gene coding for a MarR-type regulator CrtR with a nucleotide sequence according to SEQ ID NO: 14 or according to a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 14.

Preferably in the method according to the present invention, the one or more producer organism(s) added in step (ii) do/does not comprise a MarR-type regulator CrtR with an amino acid sequence according to SEQ ID NO: 15 or according to an amino acid sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 15.

Preferably in the method according to the present invention, the one or more producer organism(s) added in step (ii) is a strain of *Corynebacterium glutamicum,* which does not comprise a gene coding for a MarR-type regulator CrtR with a nucleotide sequence according to SEQ ID NO: 14 or according to a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably

at least 96%, preferably at least 97%, preferably at least 98%, preferably at least 99% sequence identity to the nucleotide sequence according to SEQ ID No: 14 and which does not comprise a MarR-type regulator CrtR with an amino acid sequence according to SEQ ID NO: 15 or according to an amino acid sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 15.

Advantageously, it was found that by using a strain of *Corynebacterium glutamicum,* as a producer organism, which does not comprise a gene coding for a MarR-type regulator CrtR and which does not comprise a MarR-type regulator CrtR, decaprenoxanthin synthesis was enhanced 10 to 30-fold as compared to the *Corynebacterium glutamicum* wild type due to deletion of the gene coding for a MarR-type regulator CrtR.

Preferably in the method according to the present invention, the growth medium provided in step (i) additionally comprises one or more substances suitable for allowing selection of the one or more producer organism(s) based on one or more reporter gene(s), preferably one or more selectable marker(s).

Preferably in the method according to the present invention, the one or more substances suitable for allowing selection of the one or more producer organism(s) based on one or more reporter gene(s) is/are one or more antibiotic agents, preferably selected from the group consisting of kanamycin, ampicillin, geneticin, particularly preferably kanamycin.

Preferably in the method according to the present invention, the one or more substances suitable for allowing selection of the one or more producer organism(s) based on one or more reporter gene(s) is biotin also known as vitamin $B_7$ or vitamin H. Using biotin as a substance allowing selection of the one or more producer organism(s) allows for the specific selection of organisms expressing biotin auxotrophic.

Preferably in the method according to the present invention, the one or more substances suitable for allowing selection of the one or more producer organism(s) my one or more species of carotenoids produced by said one or more producer organism(s), wherein said one or more species of carotenoids allow for specifically selecting organisms based on their pigmentation.

Preferably in the method according to the present invention, the one or more substances suitable for allowing selection of the one or more producer organism(s) based on one or more reporter gene(s) is sucrose. Using sucrose as a substance suitable for allowing selection of the one or more producer organism(s) based on one or more reporter gene(s) allows for using the *sacB-sacR* gene cassette as a negative selection marker.

Preferably in the method according to the present invention, the growth medium provided in step (i) additionally comprises one or more components selected from the group consisting of magnesium sulfate (MgSO4), calcium chloride (CaCl2), biotin, protocatechuic acid, iron sulfate (FeSO4), manganese sulfate (MnSO4), zinc sulfate (ZnSO4), copper sulfate (CuSO4), and nickel chloride (NiCl2), preferably iron sulfate (FeSO4), manganese sulfate (MnSO4), zinc sulfate (ZnSO4), copper sulfate (CuSO4), and nickel chloride (NiCl2), preferably iron sulfate (FeSO4), manganese sulfate (MnSO4), and zinc sulfate (ZnSO4), particularly preferably iron sulfate (FeSO4) and manganese sulfate (MnSO4).

Surprisingly, it was found that when using *Corynebacterium glutamicum* as the one or more producer organism(s) added in step (ii), the final cell dry weight obtained could be increased when increasing the amount of magnesium sulfate in the growth medium provided in step (i).

The term "magnesium sulfate" as used herein preferably refers to a chemical compound, a salt with the formula $MgSO_4$ as well as to a hydrate form of $MgSO_4$ with the chemical formula $MgSO_4{\cdot}_nH_2O$, wherein n can be any value selected from 1 (for monohydrate), 2 (for dihydrate), 3 (for trihydrate), 4 (for tetrahydrate), 5 (for pentahydrate), 6 (for hexahydrate), and 7 (for heptahydrate). Therefore, the term "magnesium sulfate" as used herein preferably encompasses anhydrous magnesium sulfate ($MgSO_4$; CAS Number 7487-88-9), magnesium sulfate monohydrate ($MgSO_4{\cdot}H_2O$; CAS Number 14168-73-1), magnesium sulfate dihydrate ($MgSO_4{\cdot}_2H_2O$), magnesium sulfate trihydrate ($MgSO_4{\cdot}_3H_2O$), magnesium sulfate tetrahydrate ($MgSO_4{\cdot}_4H_2O$; CAS Number 24378-31-2), magnesium sulfate pentahydrate ($MgSO_4{\cdot}_5H_2O$; CAS Number 15553-21-6), magnesium sulfate hexahydrate ($MgSO_4{\cdot}_6H_2O$; CAS Number 13778-97-7), magnesium sulfate heptahydrate ($MgSO_4{\cdot}_7H_2O$; CAS Number 10034-99-8). The term "magnesium sulfate" as used herein preferably encompasses the term "Epsom salt", "English salt", "bitter salt", and "bath salt".

The term "calcium chloride" as used herein preferably refers to an inorganic compound, a salt with the chemical formula $CaCl_2$ as well as to a hydrate form of $CaCl_2$ with the chemical formula $CaCl_2{\cdot}_nH_2O$, wherein n can be any value selected from 1 (for monohydrate), 2 (for dihydrate), , 4 (for tetrahydrate), and 6 (for hexahydrate). Therefore, the term "calcium chloride" as used herein preferably encompasses anhydrous calcium chloride ($CaCl_2$; CAS Number 10043-52-4), calcium chloride monohydrate ($CaCl_2{\cdot}H_2O$; CAS Number 22691-02-7), calcium chloride dihydrate ($CaCl_2{\cdot}_2H_2O$; CAS Number 10035-04-8), calcium chloride tetrahydrate ($CaCl_2{\cdot}_4H_2O$; CAS Number 25094-02-4), calcium

chloride hexahydrate ($CaCl_2 \cdot {}_6H_2O$; CAS Number 7774-34-7). The term "calcium chloride" as used herein preferably encompasses the terms "neutral calcium chloride", "calcium(II) chloride", "calcium dichloride (1 :2)", and "E509".

The term "biotin" as used herein preferably refers to a heterocyclic compound, with a sulfur-containing tetrahydrothiophene ring fused to a ureido group with the CAS Number 58-85-5, which is also known as vitamin $B_7$ or vitamin H.

The term "protocatechuic acid" as used herein preferably refers to a dihydroxybenzoic acid, a type of phenolic acid with the chemical formula $C_7H_6O_4$ and the CAS Number 99-50-3. The term "protocatechuic acid" as used herein preferably encompasses 3,4-Dihydroxybenzoic acid, PCA, and Protocatechuate.

The term "iron sulfate" as used herein preferably refers to a range of salts with the formula $FeSO_4 \cdot {}_nH_2O$, wherein n can be any value selected from 0 (for anhydrous), 1 (for monohydrate), 2 (for dihydrate), 3 (for trihydrate), 4 (for tetrahydrate), 5 (for pentahydrate), 6 (for hexahydrate), and 7 (for heptahydrate). Therefore, the term "iron sulfate" as used herein preferably refers to anhydrous iron sulfate ($FeSO_4$; CAS Number 7720-78-7), iron sulfate monohydrate ($FeSO_4 \cdot H_2O$; CAS Number 17375-41-6), iron sulfate dihydrate ($FeSO_4 \cdot {}_2H_2O$; CAS Number 10028-21-4), iron sulfate trihydrate ($FeSO_4 \cdot {}_3H_2O$), iron sulfate tetrahydrate ($FeSO_4 \cdot {}_4H_2O$), iron sulfate pentahydrate ($FeSO_4 \cdot {}_5H_2O$), iron sulfate hexahydrate ($FeSO_4 \cdot {}_6H_2O$), and iron sulfate heptahydrate ($FeSO_4 \cdot {}_7H_2O$; CAS Number 7782-63-0). The term "iron sulfate" as used herein preferably encompasses Iron(II) sulfate, Iron(II) sulfate, ferrous sulfate, green vitriol, iron vitriol, ferrous vitriol, copperas, melanterite, szomolnokite.

The "term manganese" sulfate as used herein preferably refers to the inorganic compound with the formula $MnSO_4 \cdot {}_nH_2O$ wherein n can be any value selected from 0 (for anhydrous), 1 (for monohydrate), 2 (for dihydrate), 3 (for trihydrate), and 4 (for tetrahydrate). Therefore, the term "manganese sulfate" as used herein preferably refers to anhydrous manganese sulfate ($MnSO_4$; CAS Number 7785-87-7), manganese sulfate monohydrate ($MnSO_4 \cdot H_2O$; CAS Number 10034-96-5), manganese sulfate dihydrate ($MnSO_4 \cdot {}_2H_2O$); manganese sulfate trihydrate ($MnSO_4 \cdot {}_3H_2O$), and manganese sulfate tetrahydrate ($MnSO_4 \cdot {}_4H_2O$; CAS Number 10101-68-5). The term "manganese sulfate" as used herein preferably encompasses Manganese(II) sulfate.

The term "zinc sulfate" as used herein preferably refers to a family of inorganic compounds with the formula $ZnSO_4(H_2O)_n$, wherein n can be any value selected from 1 (for monohydrate), 2 (for dihydrate), 3 (for trihydrate), 4 (for tetrahydrate), 5 (for pentahydrate), 6 (for hexahydrate), and 7 (for heptahydrate). Therefore, the term "zinc sulfate" as used herein refers to anhydrous zinc sulfate ($ZnSO_4$; CAS Number 7733-02-0), zinc sulfate monohydrate ($ZnSO_4(H_2O)$; CAS Number 7446-19-7), zinc sulfate dihydrate ($ZnSO_4(H_2O)_2$), zinc sulfate trihydrate ($ZnSO_4(H_2O)_3$), zinc sulfate tetrahydrate ($ZnSO_4(H_2O)_4$), zinc sulfate pentahydrate ($ZnSO_4(H_2O)_5$), zinc sulfate hexahydrate ($ZnSO_4(H_2O)_6$; CAS Number 13986-24-8), zinc sulfate heptahydrate ($ZnSO_4(H_2O)_7$; CAS Number 7446-20-0). The term "zinc sulfate" as used herein preferably encompasses white vitriol and goslarite.

The term "copper sulfate" as used herein preferably refers to an inorganic compound with the chemical formula $CuSO_4$ and its hydrate forms with the formula $CuSO_4 \cdot {}_nH_2O$, wherein n can be any value selected from 1 (for monohydrate), 2 (for dihydrate), 3 (for trihydrate), 4 (for tetrahydrate), 5 (for pentahydrate), 6 (for hexahydrate), and 7 (for heptahydrate). Therefore, the term "copper sulfate" as used herein preferably refers to anhydrous copper sulfate ($CuSO_4$; CAS Number 7758-98-7), copper sulfate monohydrate ($CuSO_4 \cdot H_2O$), copper sulfate dihydrate ($CuSO_4 \cdot {}_2H_2O$), copper sulfate trihydrate ($CuSO_4 \cdot {}_3H_2O$; CAS Number 16448-28-5), copper sulfate tetrahydrate ($CuSO_4 \cdot {}_4H_2O$), copper sulfate pentahydrate ($CuSO_4 \cdot {}_5H_2O$; CAS Number 7758-98-7), copper sulfate hexahydrate ($CuSO_4 \cdot {}_6H_2O$), and copper sulfate heptahydrate ($CuSO_4 \cdot {}_7H_2O$; CAS Number 19086-18-1). The term "copper sulfate" as used herein preferably encompasses copper(II) sulfate, cupric sulfate, blue vitriol (pentahydrate), bluestone (pentahydrate), bonattite (trihydrate mineral), boothite (heptahydrate mineral), chalcanthite (pentahydrate mineral), chalcocyanite (mineral), and Copper Sulfate pentahydrate.

The term "nickel chloride" as used herein preferably refers to a chemical compound with the formula $NiCl_2$ as well as to any hydrate form of said compound with the formula $NiCl_2 \cdot {}_nH_2O$, wherein n can be any value selected from 1 (for monohydrate), 2 (for dihydrate), 3 (for trihydrate), 4 (for tetrahydrate), 5 (for pentahydrate), and 6 (for hexahydrate). n can be any value selected from 1 (for monohydrate), 2 (for dihydrate), 3 (for trihydrate), 4 (for tetrahydrate), 5 (for pentahydrate), 6 (for hexahydrate). Therefore, the term "nickel chloride" as used herein preferably refers to anhydrous nickel chloride ($NiCl_2$; CAS Number 7718-54-9) nickel chloride monohydrate ($NiCl_2 \cdot H_2O$), nickel chloride dihydrate ($NiCl_2 \cdot {}_2H_2O$), nickel chloride trihydrate ($NiCl_2 \cdot {}_3H_2O$), nickel chloride tetrahydrate ($NiCl_2 \cdot {}_4H_2O$), nickel chloride pentahydrate ($NiCl_2 \cdot {}_5H_2O$), nickel chloride hexahydrate ($NiCl_2 \cdot {}_6H_2O$; CAS Number 7791-20-0). The term "nickel chloride" as used herein preferably encompasses nickel(II) chloride, nickelous chloride, nickel(II) salt of hydrochloric acid.

Preferably in the method according to the present invention, the growth medium provided in step (i) additionally comprises iron sulfate heptahydrate ($FeSO_4 \cdot {}_7H_2O$; CAS Number 7782-63-0), manganese sulfate monohydrate ($MnSO_4 \cdot H_2O$; CAS Number 10034-96-5), zinc sulfate heptahydrate ($ZnSO_4(H_2O)_7$; CAS Number 7446-20-0), copper sulfate ($CuSO_4$; CAS Number 7758-98-7), and nickel chloride hexahydrate ($NiCl_2 \cdot {}_6H_2O$; CAS Number 7791-20-0).

Preferably in the method according to the present invention, the growth medium provided in step (i) additionally comprises iron sulfate heptahydrate ($FeSO_4 \cdot {}_7H_2O$; CAS Number 7782-63-0), manganese sulfate monohydrate

($MnSO_4 \cdot H_2O$; CAS Number 10034-96-5), zinc sulfate heptahydrate ($ZnSO_4(H_2O)_7$; CAS Number 7446-20-0), copper sulfate ($CuSO_4$; CAS Number 7758-98-7), nickel chloride hexahydrate ($NiCl_2 \cdot {}_6H_2O$; CAS Number 7791-20-0), biotin (CAS Number 58-85-5), protocatechuic acid ($C_7H_6O_4$; CAS Number 99-50-3), magnesium sulfate heptahydrate ($MgSO_4 \cdot {}_7H_2O$; CAS Number 10034-99-8), and calcium chloride dihydrate ($CaCl_2 \cdot {}_2H_2O$; CAS Number 10035-04-8).

Preferably in the method according to the present invention, the growth medium provided in step (i) additionally comprises iron sulfate heptahydrate ($FeSO_4 \cdot {}_7H_2O$; CAS Number 7782-63-0), manganese sulfate monohydrate ($MnSO_4 \cdot H_2O$; CAS Number 10034-96-5), zinc sulfate heptahydrate ($ZnSO_4(H_2O)_7$; CAS Number 7446-20-0), copper sulfate ($CuSO_4$; CAS Number 7758-98-7), nickel chloride hexahydrate ($NiCl_2 \cdot {}_6H_2O$; CAS Number 7791-20-0), biotin (CAS Number 58-85-5), protocatechuic acid ($C_7H_6O_4$; CAS Number 99-50-3), magnesium sulfate heptahydrate ($MgSO_4 \cdot {}_7H_2O$; CAS Number 10034-99-8), and calcium chloride dihydrate ($CaCl_2 \cdot {}_2H_2O$; CAS Number 10035-04-8) and the one or more producer organism(s) added in step (ii) is *Corynebacterium glutamicum.*

Preferably in the method according to the present invention, the growth medium provided in step (i) additionally comprises iron sulfate heptahydrate ($FeSO_4 \cdot {}_7H_2O$; CAS Number 7782-63-0) at a concentration in the range of from 11.5 to 28.5 mg/L, preferably of from 15.0 to 23.0 mg/L, preferably of from 19 to 20 mg/L, particularly preferably of 20 mg/L.

Preferably in the method according to the present invention, the growth medium provided in step (i) additionally comprises iron sulfate heptahydrate ($FeSO_4 \cdot {}_7H_2O$; CAS Number 7782-63-0) at a concentration in the range of from 11.5 to 28.5 mg/L, preferably of from 15.0 to 23.0 mg/L, preferably of from 19 to 20 mg/L, particularly preferably of 20 mg/L and the one or more producer organism(s) added in step (ii) is *Corynebacterium glutamicum..*

Surprisingly, it was found that when adjusting the concentration of iron sulfate in the growth medium provided in step (i) according to the present invention as described herein in combination with using *Corynebacterium glutamicum* as the one or more producer organism(s) the overall titer of carotenoids produced could be increased.

Particularly surprisingly, it was found that when adjusting the concentration of iron sulfate in the growth medium provided in step (i) according to the present invention as described herein in combination with using *Corynebacterium glutamicum* as the one or more producer organism(s) the titer of astaxanthin produced either alone or in combination with one or more other species of carotenoids could be increased.

Preferably in the method according to the present invention, the growth medium provided in step (i) additionally comprises manganese sulfate monohydrate ($MnSO_4 \cdot H_2O$; CAS Number 10034-96-5) at a concentration in the range of from 0.5 mg/L to 10.0 mg/L, preferably of from 0.6 mg/L to 9.5 mg/L, preferably of from 0.7 mg/L to 9.3 mg/L, preferably of from 0.8 mg/L to 9.2 mg/L, preferably of from 0.84 mg/L to 9.2 mg/L, particularly preferably of 5 mg/L.

Preferably in the method according to the present invention, the growth medium provided in step (i) additionally comprises manganese sulfate monohydrate ($MnSO_4 \cdot H_2O$; CAS Number 10034-96-5) at a concentration in the range of from 0.5 mg/L to 10.0 mg/L, preferably of from 0.6 mg/L to 9.5 mg/L, preferably of from 0.7 mg/L to 9.3 mg/L, preferably of from 0.8 mg/L to 9.2 mg/L, preferably of from 0.84 mg/L to 9.2 mg/L, particularly preferably of 5 mg/L and the one or more producer organism(s) added in step (ii) is *Corynebacterium glutamicum.*

Surprisingly, it was found that when adjusting the concentration of manganese sulfate in the growth medium provided in step (i) according to the present invention as described herein in combination with using *Corynebacterium glutamicum* as the one or more producer organism(s) the overall titer of carotenoids produced could be increased.

Particularly surprisingly, it was found that when adjusting the concentration of manganese sulfate in the growth medium provided in step (i) according to the present invention as described herein in combination with using *Corynebacterium glutamicum* as the one or more producer organism(s) the titer of astaxanthin produced either alone or in combination with one or more other species of carotenoids could be increased.

Preferably in the method according to the present invention, the growth medium provided in step (i) additionally comprises zinc sulfate heptahydrate ($ZnSO_4(H_2O)_7$; CAS Number 7446-20-0) at a concentration in the range of from 1.0 to 1.4 mg/L, preferably of from 1.1 to 1.3 mg/L, particularly preferably of from 1.1 to 1.2 mg/L.

Preferably in the method according to the present invention, the growth medium provided in step (i) additionally comprises zinc sulfate heptahydrate ($ZnSO_4(H_2O)_7$; CAS Number 7446-20-0) at a concentration in the range of from 1.0 to 1.4 mg/L, preferably of from 1.1 to 1.3 mg/L, particularly preferably of from 1.1 to 1.2 mg/L and the one or more producer organism(s) added in step (ii) is *Corynebacterium glutamicum.*

Surprisingly, it was found that when adjusting the concentration of zinc sulfate in the growth medium provided in step (i) according to the present invention as described herein in combination with using *Corynebacterium glutamicum* as the one or more producer organism(s) the overall titer of carotenoids produced could be increased.

Particularly surprisingly, it was found that when adjusting the concentration of zinc sulfate in the growth medium provided in step (i) according to the present invention as described herein in combination with using *Corynebacterium glutamicum* as the one or more producer organism(s) the titer of astaxanthin produced either alone or in combination with one or more other species of carotenoids could be increased.

Preferably in the method according to the present invention, the growth medium provided in step (i) additionally comprises copper sulfate ($CuSO_4$; CAS Number 7758-98-7) at a concentration in the range of from 0.0015 to 0.2 mg/L, preferably of from 0.05 to 0.15 mg/L, particularly preferably of from 0.09 to 0.1 mg/L.

Preferably in the method according to the present invention, the growth medium provided in step (i) additionally comprises copper sulfate ($CuSO_4$; CAS Number 7758-98-7) at a concentration in the range of from 0.0015 to 0.2 mg/L, preferably of from 0.05 to 0.15 mg/L, particularly preferably of from 0.09 to 0.1 mg/L and the one or more producer organism(s) added in step (ii) is *Corynebacterium glutamicum.*

Surprisingly, it was found that when adjusting the concentration of copper sulfate in the growth medium provided in step (i) according to the present invention as described herein in combination with using *Corynebacterium glutamicum* as the one or more producer organism(s) the overall titer of carotenoids produced could be increased.

Particularly surprisingly, it was found that when adjusting the concentration of copper sulfate in the growth medium provided in step (i) according to the present invention as described herein in combination with using *Corynebacterium glutamicum* as the one or more producer organism(s) the titer of astaxanthin produced either alone or in combination with one or more other species of carotenoids could be increased.

Preferably in the method according to the present invention, the growth medium provided in step (i) additionally comprises nickel chloride hexahydrate ($NiCl_2 \cdot {}_6H_2O$; CAS Number 7791-20-0) at a concentration in the range of from 0.12 to 0.16 mg/L, preferably of from 0.13 to 0.15 mg/L, particularly preferably of from 0.14 to 0.15 mg/L.

Preferably in the method according to the present invention, the growth medium provided in step (i) additionally comprises nickel chloride hexahydrate ($NiCl_2 \cdot {}_6H_2O$; CAS Number 7791-20-0) at a concentration in the range of from 0.12 to 0.16 mg/L, preferably of from 0.13 to 0.15 mg/L, particularly preferably of from 0.14 to 0.15 mg/L and the one or more producer organism(s) added in step (ii) is *Corynebacterium glutamicum.*

Surprisingly, it was found that when adjusting the concentration of nickel chloride in the growth medium provided in step (i) according to the present invention as described herein in combination with using *Corynebacterium glutamicum* as the one or more producer organism(s) the overall titer of carotenoids produced could be increased.

Particularly surprisingly, it was found that when adjusting the concentration of nickel chloride in the growth medium provided in step (i) according to the present invention as described herein in combination with using *Corynebacterium glutamicum* as the one or more producer organism(s) the titer of astaxanthin produced either alone or in combination with one or more other species of carotenoids could be increased.

Preferably in the method according to the present invention, the growth medium provided in step (i) additionally comprises iron sulfate heptahydrate ($FeSO_4 \cdot {}_7H_2O$; CAS Number 7782-63-0) at a concentration in the range of from 11.5 to 28.5 mg/L, preferably of from 15.0 to 23.0 mg/L, particularly preferably of from 19 to 20 mg/L, and manganese sulfate monohydrate ($MnSO_4 \cdot H_2O$; CAS Number 10034-96-5) at a concentration in the range of from 0.5 mg/L to 10.0 mg/L, preferably of from 0.6 mg/L to 9.7 mg/L, preferably of from 0.7 mg/L to 9.5 mg/L, preferably of from 0.8 mg/L to 9.3 mg/L, preferably of from 0.84 mg/L to 9.2 mg/L, preferably of from 1.0 mg/L to 8.0 mg/L, preferably of from 2.0 mg/L to 7.0 mg/L, particularly preferably of 5 mg/L, and zinc sulfate heptahydrate ($ZnSO_4(H_2O)_7$; CAS Number 7446-20-0) at a concentration in the range of from 1.0 to 1.4 mg/L, preferably of from 1.1 to 1.3 mg/L, particularly preferably of from 1.1 to 1.2 mg/L, and copper sulfate ($CuSO_4$; CAS Number 7758-98-7) at a concentration in the range of from 0.0015 to 0.2 mg/L, preferably of from 0.05 to 0.15 mg/L, particularly preferably of from 0.09 to 0.1 mg/L, and nickel chloride hexahydrate ($NiCl_2 \cdot {}_6H_2O$; CAS Number 7791-20-0) at a concentration in the range of from 0.12 to 0.16 mg/L, preferably of from 0.13 to 0.15 mg/L, particularly preferably of from 0.14 to 0.15 mg/L.

Preferably in the method according to the present invention, the growth medium provided in step (i) additionally comprises iron sulfate heptahydrate ($FeSO_4 \cdot {}_7H_2O$; CAS Number 7782-63-0) at a concentration in the range of from 11.5 to 28.5 mg/L, preferably of from 15.0 to 23.0 mg/L, particularly preferably of from 19 to 20 mg/L, and manganese sulfate monohydrate ($MnSO_4 \cdot H_2O$; CAS Number 10034-96-5) at a concentration in the range of from 0.5 mg/L to 10.0 mg/L, preferably of from 0.6 mg/L to 9.5 mg/L, preferably of from 0.7 mg/L to 9.3 mg/L, preferably of from 0.8 mg/L to 9.2 mg/L, preferably of from 0.84 mg/L to 9.2 mg/L, particularly preferably of 5 mg/L, and zinc sulfate heptahydrate ($ZnSO_4(H_2O)_7$; CAS Number 7446-20-0) at a concentration in the range of from 1.0 to 1.4 mg/L, preferably of from 1.1 to 1.3 mg/L, particularly preferably of from 1.1 to 1.2 mg/L, and copper sulfate ($CuSO_4$; CAS Number 7758-98-7) at a concentration in the range of from 0.0015 to 0.2 mg/L, preferably of from 0.05 to 0.15 mg/L, particularly preferably of from 0.09 to 0.1 mg/L, and nickel chloride hexahydrate ($NiCl_2 \cdot {}_6H_2O$; CAS Number 7791-20-0) at a concentration in the range of from 0.12 to 0.16 mg/L, preferably of from 0.13 to 0.15 mg/L, particularly preferably of from 0.14 to 0.15 mg/L and the one or more producer organism(s) added in step (ii) is *Corynebacterium glutamicum.*

Surprisingly, it was found that when adjusting the concentration of iron sulfate, manganese sulfate, zinc sulfate, copper sulfate, and nickel chloride in the growth medium provided in step (i) according to the present invention as described herein in combination with using *Corynebacterium glutamicum* as the one or more producer organism(s) the overall titer of carotenoids produced could be increased.

Particularly surprisingly, it was found that when adjusting the concentration of iron sulfate, manganese sulfate, zinc sulfate, copper sulfate, and nickel chloride in the growth medium provided in step (i) according to the present invention as described herein in combination with using *Corynebacterium glutamicum* as the one or more producer organism(s) the titer of astaxanthin produced either alone or in combination with one or more other species of carotenoids could be increased to a range of from 30 to 36 mg/L.

Particularly surprisingly, it was found that when adjusting the concentration of iron sulfate, manganese sulfate, zinc sulfate, copper sulfate, and nickel chloride in the growth medium provided in step (i) according to the present invention as described herein in combination with using *Corynebacterium glutamicum* as the one or more producer organism(s) the overall titer of carotenoids produced could be increased to a range of from 50 to 85 mg/L.

Preferably in the method according to the present invention, the growth medium provided in step (i) comprises zinc at a concentration in the range of from 0.1 to 0.4 mg/L, preferably of from 0.15 to 0.35 mg/L, preferably of from 0.2 to 0.3 mg/L, preferably of from 0.25 to 0.28 mg/L particularly preferably of from 0.26 to 0.27 mg/L, and nickel at a concentration in the range of from 0.02 to 0.05 mg/L, preferably of from 0.025 to 0.04 mg/L, preferably of from 0.03 to 0.038 mg/L particularly preferably of from 0.035 to 0.036 mg/L, and iron at a concentration in the range of from 3.0 to 5.0 mg/L, preferably of from 3.5 to 4.5 mg/L, particularly preferably of from 4.0 to 4.1 mg/L, and manganese at a concentration in the range of from 1.4 to 2.0 mg/L, preferably of from 1.5 to 1.8 mg/L, particularly preferably of from 1.6 to 1.7 mg/L, and copper at a concentration in the range of from 0.0015 to 0.1 mg/L, preferably of from 0.02 to 0.05 mg/L, preferably of from 0.03 to 0.045 mg/L, particularly preferably of from 0.39 to 0.04 mg/L.

Preferably in the method according to the present invention, the growth medium provided in step (i) comprises zinc at a concentration in the range of from 0.1 to 0.4 mg/L, preferably of from 0.15 to 0.35 mg/L, preferably of from 0.2 to 0.3 mg/L, preferably of from 0.25 to 0.28 mg/L particularly preferably of from 0.26 to 0.27 mg/L, and nickel at a concentration in the range of from 0.02 to 0.05 mg/L, preferably of from 0.025 to 0.04 mg/L, preferably of from 0.03 to 0.038 mg/L particularly preferably of from 0.035 to 0.036 mg/L, and iron at a concentration in the range of from 3.0 to 5.0 mg/L, preferably of from 3.5 to 4.5 mg/L, particularly preferably of from 4.0 to 4.1 mg/L, and manganese at a concentration in the range of from 1.4 to 2.0 mg/L, preferably of from 1.5 to 1.8 mg/L, particularly preferably of from 1.6 to 1.7 mg/L, and copper at a concentration in the range of from 0.0015 to 0.1 mg/L, preferably of from 0.02 to 0.05 mg/L, preferably of from 0.03 to 0.045 mg/L, particularly preferably of from 0.39 to 0.04 mg/L, wherein the amounts given in mg/L refer to the respective metal ions.

Preferably in the method according to the present invention, the growth medium provided in step (i) comprises zinc at a concentration in the range of from 0.1 to 0.4 mg/L, preferably of from 0.15 to 0.35 mg/L, preferably of from 0.2 to 0.3 mg/L, preferably of from 0.25 to 0.28 mg/L particularly preferably of from 0.26 to 0.27 mg/L, preferably at a concentration of 0.268 mg/L.

Preferably in the method according to the present invention, the growth medium provided in step (i) comprises nickel at a concentration in the range of from 0.02 to 0.05 mg/L, preferably of from 0.025 to 0.04 mg/L, preferably of from 0.03 to 0.038 mg/L particularly preferably of from 0.035 to 0.036 mg/L, preferably at a concentration of 0.0356 mg/L.

Preferably in the method according to the present invention, the growth medium provided in step (i) comprises iron at a concentration in the range of from 3.0 to 5.0 mg/L, preferably of from 3.5 to 4.5 mg/L, particularly preferably of from 4.0 to 4.1 mg/L, preferably at a concentration of 4.018 mg/L.

Preferably in the method according to the present invention, the growth medium provided in step (i) comprises manganese at a concentration in the range of from 1.4 to 2.0 mg/L, preferably of from 1.5 to 1.8 mg/L, particularly preferably of from 1.6 to 1.7 mg/L, preferably at a concentration of 1.625 mg/L.

Preferably in the method according to the present invention, the growth medium provided in step (i) comprises copper at a concentration in the range of from 0.0015 to 0.1 mg/L, preferably of from 0.02 to 0.05 mg/L, preferably of from 0.03 to 0.045 mg/L, particularly preferably of from 0.39 to 0.04 mg/L, preferably at a concentration of 0.0398 mg/L.

Surprisingly, it was found that when adjusting the concentration of iron or iron sulfate, manganese or manganese sulfate, zinc or zinc sulfate, copper or copper sulfate, and nickel or nickel chloride in the growth medium provided in step (i) according to the present invention as described herein in combination with adjusting the concentration of potassium acetate, glucose, ammonium sulfate, urea, and phosphate in the growth medium provided in step (i) according to the present invention as described herein and in combination with using *Corynebacterium glutamicum* as the one or more producer organism(s) the overall titer of carotenoids produced could be increased.

Surprisingly, it was found that when adjusting the concentration of iron or iron sulfate, manganese or manganese sulfate, zinc or zinc sulfate, copper or copper sulfate, and nickel or nickel chloride in the growth medium provided in step (i) according to the present invention as described herein in combination with adjusting the concentration of potassium acetate, glucose, ammonium sulfate, urea, and phosphate in the growth medium provided in step (i) according to the present invention as described herein and in combination with using *Corynebacterium glutamicum* as the one or more producer organism(s) the purity of astaxanthin produced could be increased.

Particularly surprisingly, it was found that when adjusting the concentration of iron or iron or iron sulfate, manganese or manganese sulfate, zinc or zinc sulfate, copper or copper sulfate, and nickel or nickel chloride in the growth medium provided in step (i) according to the present invention as described herein in combination with adjusting the concentration of potassium acetate, glucose, ammonium sulfate, urea, and phosphate in the growth medium provided in step (i) according to the present invention as described herein and in combination with using *Corynebacterium glutamicum* as the one or more producer organism(s) the overall titer of carotenoids produced could be increased to a range of from 55 to 90 mg/L.

Particularly surprisingly, it was found that when adjusting the concentration of iron or iron or iron sulfate,

manganese or manganese sulfate, zinc or zinc sulfate, copper or copper sulfate, and nickel or nickel chloride in the growth medium provided in step (i) according to the present invention as described herein in combination with adjusting the concentration of potassium acetate to 12.6 to 13 g/L, the concentration of glucose to a range of from 48 ton 52 g/L, the concentration of ammonium sulfate to a range of from 9.5 to 10.5 g/L, the concentration of urea to a range of from 7 to 8 g/L, and the concentration of phosphate to a range of from 1.5 to 2.5 g/L in the growth medium provided in step (i) according to the present invention as described herein and in combination with using *Corynebacterium glutamicum* as the one or more producer organism(s) the overall titer of carotenoids produced could be increased to approximately 85 mg/L while the titer of astaxanthin produced could be increased to approximately 35 mg/L.

Particularly surprisingly, it was found that when adjusting the concentration of iron or iron or iron sulfate, manganese or manganese sulfate, zinc or zinc sulfate, copper or copper sulfate, and nickel or nickel chloride in the growth medium provided in step (i) according to the present invention as described herein in combination with adjusting the concentration of potassium acetate to 12.6 to 13 g/L, the concentration of glucose to a range of from 48 ton 52 g/L, the concentration of ammonium sulfate to a range of from 9.5 to 10.5 g/L, the concentration of urea to a rage of from 7 to 8 g/L, and the concentration of phosphate to a range of from 1.5 to 2.5 g/L in the growth medium provided in step (i) according to the present invention as described herein and in combination with using *Corynebacterium glutamicum* as the one or more producer organism(s) the overall titer of carotenoids produced could be increased to approximately 55 g/L while the titer of astaxanthin produced could be increased to approximately 34 g/L.

Therefore, the method of the present invention allows for producing (i) a high overall titer of different species of carotenoids, particularly of astaxanthin while the method of the present invention also allows for producing a high overall titer of different species of carotenoids, wherein astaxanthin makes up more than 50%, even more than 60%, of the overall produced species of carotenoids.

Preferably in the method according to the present invention, the or, respectively, one of the one or more producer organism(s) is a strain of *Corynebacterium glutamicum* or a naturally occurring or artificially generated mutant thereof, preferably wherein said strain of *Corynebacterium glutamicum* comprises at least one 16S rRNA molecule, which is encoded by a DNA sequence according to SEQ ID No: 16 or a DNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the DNA sequence according to SEQ ID No: 16.

Preferably in the method according to the present invention, the or, respectively, one of the one or more producer organism(s) is a strain of *Corynebacterium glutamicum* or a naturally occurring or artificially generated mutant thereof, preferably wherein said strain of *Corynebacterium glutamicum* comprises at least one 16S rRNA molecule, which has an RNA sequence according to SEQ ID No: 17 or an RNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the RNA sequence according to SEQ ID No: 17.

It was found that using a strain of *Corynebacterium glutamicum* or a naturally occurring or artificially generated mutant thereof as the one of the one or more producer organism(s) in a method according to the present invention, which comprises at least one 16S rRNA molecule, which is encoded by a DNA sequence according to SEQ ID No: 16 or a DNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the DNA sequence according to SEQ ID No: 16 and/or at least one 16S rRNA molecule, which has an RNA sequence according to SEQ ID No: 17 or an RNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the RNA sequence according to SEQ ID No: 17, allowed for a particularly high biomass formation of the one of the one or more producer organism(s) and thus for particularly resource-efficient, cost-efficient, energy-efficient, and sustainable method for producing one or more species of carotenoids, particularly astaxanthin.

It was also found that using a strain of *Corynebacterium glutamicum* or a naturally occurring or artificially generated mutant thereof as the one of the one or more producer organism(s) in a method according to the present invention, which comprises at least one 16S rRNA molecule, which is encoded by a DNA sequence according to SEQ ID No: 16 or a DNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the DNA sequence according to SEQ ID No: 16 and/or at least one 16S rRNA

molecule, which has an RNA sequence according to SEQ ID No: 17 or an RNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the RNA sequence according to SEQ ID No: 17, allowed for a particularly high biomass formation rate (i.e. growth rate) of the one of the one or more producer organism(s) and thus for particularly cost-efficient, energy-efficient, and sustainable method for producing one or more species of carotenoids, particularly astaxanthin.

Preferably in the method according to the present invention, the growth medium provided in step (i) additionally comprises one or more solidifying agent(s), preferably selected from the group consisting of agar, agarose, alginate, and carrageenan, preferably agar.

The term "agar" as used herein preferably refers to a substance comprising or consisting of polysaccharides obtained from the cell walls of some species of red algae, primarily from "*ogonon*" (*Gracilaria*) and "*tengusa*" (*Gelidiaceae*). The term "agar" as used herein preferably encompasses the term "agar-agar". Preferably, the terms "agar" and "agar-agar" can be used interchangeably.

The term "agarose" as used herein refers to a heteropolysaccharide, generally extracted from red seaweed, which is a linear polymer made up of the repeating unit of agarobiose, which is a disaccharide made up of D-galactose and 3,6-anhydro-L-galactopyranose.

Advantageously, using a solidifying agent allows for the growth medium provided in step (i) of the method according to the present invention allows for reversibly solidifying and de-solidifying said growth medium.

Thus, the growth medium provided in step (i) of the method according to the present invention is suitable for use in a solid state, e.g. in the form of agar plates and other Petri dishes, as well as in a liquid state.

In a further aspect, the present invention relates to a growth medium comprising ammonium sulfate, urea, phosphate, glucose, and potassium acetate, for production of one or more species of carotenoids via a producer organism,

> wherein the concentration ratio of glucose to potassium acetate in said composition is in the range of from 3.0 to 4.0 and
>
> the concentration ratio of glucose to ammonium sulfate in said composition is in the range of from 2.0 to 5.5 and
>
> the concentration ratio of glucose to urea in said composition is in the range of from 6.0 to 12.5 and
>
> the concentration ratio of glucose to phosphate in said composition is the range of from 11.0 to 27.0,
>
> preferably wherein the growth medium is a growth medium as defined herein for any aspect of the present invention.

What was said above with regard to the method according to the present invention applies accordingly to the growth medium according to the invention. Particularly, all the preferred features for the growth medium provided in step (i) of the method according to the present invention as described herein (preferably those embodiments described herein as preferred) are also preferred embodiments of the growth medium according to the present invention and the technical effects associated with the preferred embodiments for the growth medium provided in step (i) of the method according to the present invention as described herein also apply for the growth medium according to the present invention.

In an even further aspect, the present invention relates to a kit comprising the following components:

> (a) Growth medium comprising ammonium sulfate, urea, phosphate, glucose, and potassium acetate, for production of one or more species of carotenoids via a producer organism,
>
> > wherein the concentration ratio of glucose to potassium acetate in said medium is in the range of from 3.0 to 4.0 and
> >
> > the concentration ratio of glucose to ammonium sulfate in said medium is in the range of from 2.0 to 5.5 and
> >
> > the concentration ratio of glucose to urea in said medium is in the range of from 6.0 to 12.5 and
> >
> > the concentration ratio of glucose to phosphate in said medium is the range of from 11.0 to 27.0,
> >
> > preferably wherein the growth medium is a growth medium as defined herein for any aspect of the present invention;

(b) One or more producer organism(s), preferably one or more bacterial producer organism(s), particularly preferably one or more producer organism(s) as defined herein for any aspect of the present invention;

(c) optionally: One or more cultivation device(s) for cultivating said one or more producer organism(s) in said growth medium

(d) optionally: One or more pieces of laboratory consumables, preferably selected from the group consisting of disposable pipettes, disposable beakers, disposable flasks, disposable bottles, disposable tubes, chromatography columns, filters, spatulas, spoons;

(e) optionally: One or more pieces of laboratory equipment, preferably selected from the group consisting of glass pipettes, beakers, flasks, bottles, mixers, scales, incubators, measuring cylinders;

(f) optionally: Instructions for use.

What was said above with regard to the method according to the present invention applies accordingly to the kit according to the invention. Particularly, all the preferred embodiments for the growth medium provided in step (i) of the method according to the present invention as described herein (preferably those embodiments described herein as preferred) are also preferred embodiments of the growth medium comprised in the kit according to the present invention and the technical effects associated with the preferred embodiments for the growth medium provided in step (i) of the method according to the present invention as described herein also apply for the growth medium in the kit according to the present invention.

All the preferred embodiments for the one or more producer organism(s) used in the method according to the present invention as described herein (preferably those embodiments described herein as preferred) are also preferred embodiments of the one or more producer organism(s) comprised in the kit according to the present invention and the technical effects associated with the one or more producer organism(s) used in the method according to the present invention as described herein also apply for the growth medium in the kit according to the present invention.

Preferably in the kit according to the present invention, the one or more producer organism(s) are comprised in a state allowing for prolonged storage and/or easy transport and/or quick and easy reactivation, such as lyophilized, freeze dried, as glycerol stocks, or as colonies on agar pates and/or other Petri dishes.

Preferably in the kit according to the present invention, the one or more cultivation device(s) are selected from the group consisting of Erlenmeyer flasks, preferably in combination with one or more rotary shakers, bioreactors with and/or without one or more electric control unit(s), Petri dishes, and incubators.

Preferably in the kit according to the present invention, the kit comprises one or more pieces of laboratory consumables, preferably selected from the group consisting of disposable pipettes, disposable beakers, disposable flasks, disposable bottles, disposable tubes, chromatography columns, filters, spatulas, spoons.

Preferably in the kit according to the present invention, the kit comprises one or more pieces of laboratory equipment, preferably selected from the group consisting of glass pipettes, beakers, flasks, bottles, mixers, scales, incubators, measuring cylinders.

Thus, the kit according to the invention preferably is ready-to-use without requiring the acquisition of additional components for conducting the method according to the present invention as described herein.

In an even further aspect, the present invention relates to the use of an expression construct or a vector in a method according to the present invention, wherein the expression construct facilitates production of one or more species of carotenoids, preferably astaxanthin, lycopene, echinenone, cryptoxanthin, hydroxyechinenone, zeaxanthin, and adonirubin, preferably astaxanthin, β-carotene, and canthaxanthin, particularly preferably astaxanthin, wherein the expression constructs encodes at least one lycopene cyclase enzyme CrtY and/or a β-carotene ketolase enzyme CrtW and/or at least one β-carotene hydroxylase enzyme CrtZ,

wherein the lycopene cyclase enzyme CrtY has an amino acid sequence according to SEQ ID NO: 1 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence similarity to the amino acid sequence according to SEQ ID No: 1, and/or

wherein the β-carotene ketolase enzyme CrtW has an amino acid sequence according to SEQ ID NO: 2 or an amino acid sequence having at least 80%, preferably at least 81%, preferably at least 82%, preferably at least 83%,

preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence similarity to the amino acid sequence according to SEQ ID No: 2, and/or

wherein the β-carotene hydroxylase enzyme CrtZ has an amino acid sequence according to SEQ ID NO: 3 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence similarity to the amino acid sequence according to SEQ ID No: 3.

Preferably in the use of an expression construct or a vector in a method according to the present invention, the nucleic acid sequence encoding the lycopene cyclase enzyme CrtY and the nucleic acid sequence encoding the β-carotene ketolase enzyme CrtW and the nucleic acid sequence encoding the β-carotene hydroxylase enzyme CrtZ are present on the same expression construct or vector, or on at least two individual expression constructs or vectors.

Preferably in the use of an expression construct or a vector in a method according to the present invention, the nucleic acid sequence encoding the lycopene cyclase enzyme CrtY and/or the nucleic acid sequence encoding the β-carotene ketolase enzyme CrtW and/or the nucleic acid sequence encoding the β-carotene hydroxylase enzyme CrtZ is/are expressed from an RNA polymerase II promoter or an RNA polymerase III promoter, preferably wherein the promoter is a $P_{tuf}$ promoter, preferably corresponding to a sequence according to SEQ ID NO: 18.

It was found that expression of the nucleic acid sequence encoding the lycopene cyclase enzyme CrtY and/or the nucleic acid sequence encoding the β-carotene ketolase enzyme CrtW and/or the nucleic acid sequence encoding the β-carotene hydroxylase enzyme CrtZ from a $P_{tuf}$ promoter led to the highest expression rates.

Preferably in the use of an expression construct or a vector in a method according to the present invention, the genes coding for exogenously expressed lycopene cyclase enzyme CrtY, β-carotene ketolase enzyme CrtW, and β-carotene hydroxylase enzyme CrtZ are encoded adjacently in a combinatorial gene assembly as part of an expression construct or vector, wherein the specific order of the genes coding for exogenously expressed lycopene cyclase enzyme CrtY, β-carotene ketolase enzyme CrtW, and β-carotene hydroxylase enzyme CrtZ is variable.

Preferably in the use of an expression construct or a vector in a method according to the present invention, the genes coding for exogenously expressed lycopene cyclase enzyme CrtY, β-carotene ketolase enzyme CrtW, and β-carotene hydroxylase enzyme CrtZ are encoded adjacently in a combinatorial gene assembly as part of an expression construct or vector, and wherein the stop codon of a first gene and the start codon of an adjacently coded gene are separated by a ribosomal binding site and a spacing sequence, wherein said ribosomal binding site has a size of 9 bp and the spacing sequence has a size of 10 bp or less, preferably 8 bp or less, preferably 6 bp or less, preferably 3 bp or less. Preferably, the ribosomal binding site has a sequence according to any one of the sequences selected from the group consisting of SEQ ID NO 7, SEQ ID NO 8, SEQ ID NO 9, and SEQ ID NO 10 and/or the spacing sequence has a sequence according to any one of the sequences selected from the group consisting of SEQ ID NO 11, SEQ ID NO 12, and SEQ ID NO 13.

Preferably in the use of an expression construct or a vector in a method according to the present invention, the genes coding for exogenously expressed lycopene cyclase enzyme CrtY, β-carotene ketolase enzyme CrtW, and β-carotene hydroxylase enzyme CrtZ are encoded adjacently in a combinatorial gene assembly as part of an expression construct or vector, wherein the genes coding for exogenously expressed lycopene cyclase enzyme CrtY, β-carotene ketolase enzyme CrtW, and β-carotene hydroxylase enzyme CrtZ each comprise a start codon, which is either ATG or GTG, preferably ATG.

Advantageously, by separating the genes coding for exogenously expressed lycopene cyclase enzyme CrtY, β-carotene ketolase enzyme CrtW, and β-carotene hydroxylase enzyme CrtZ each by a ribosomal binding site and a spacing sequence according to the present invention as described herein on the expression construct or vector, the transcription initiation rates of each of these exogenously expressed enzymes could be optimized and are variably adjustable.

What was said above with regard to the method according to the invention, and/or with regard to the growth medium according to the invention, and/or with regard to the kit according to the invention applies accordingly to the use according to the invention, particularly all the preferred embodiments for the one or more producer organism(s) of the method according to the present invention as described herein (preferably those embodiments described herein as preferred) are also preferred embodiments of the cell for the use of a cell according to the present invention and the technical effects associated with the preferred embodiments for the one or more producer organism(s) of the method according to the present invention as described herein also apply for the cell for the use of a cell according to the present invention.

In an even further aspect, the present invention relates to the use of a cell in a method according to the present invention, wherein the cell comprises an expression construct or a vector as described herein, preferably wherein said cell is a *Corynebacterium glutamicum* cell and comprises at least one 16S rRNA molecule, which is encoded by a DNA sequence according to SEQ ID No: 16 or a DNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the DNA sequence according to SEQ ID No: 16.

What was said above with regard to the method according to the invention, and/or with regard to the growth medium according to the invention, and/or with regard to the kit according to the invention applies accordingly to the use according to the invention, particularly all the preferred embodiments for the one or more producer organism(s) of the method according to the present invention as described herein (preferably those embodiments described herein as preferred) are also preferred embodiments of the cell for the use of a cell according to the present invention and the technical effects associated with the preferred embodiments for the one or more producer organism(s) of the method according to the present invention as described herein also apply for the cell for the use of a cell according to the present invention.

## Examples

**Example 1:** Producer organism and cultivation conditions

In order to identify important media components and to increase astaxanthin titers, a design of experiment (DOE) approach was followed. As a producer organism *Corynebacterium glutamicum* strain ASTA* (cf. Henke, N. A.; Wendisch, V. F. Improved Astaxanthin Production with Corynebacterium Glutamicum by Application of a Membrane Fusion Protein. Mar. Drugs 2019, 17, doi: 10.3390/md17110621), a derivative of strain BETA4 carrying pSH1 ::crtZ-WFP plasmid to allow for astaxanthin production, was used.

All cultivations of this strain contained 25 μg/mL kanamycin. Pre-cultures were first performed on LB agar plates. These were incubated for 48 h at 30 °C and in darkness, after which a single colony was used to inoculate 10 mL LB liquid medium with 1% (w/v) of glucose added. This first pre-culture was incubated on a rotary incubator for 20 h at 30 °C in the dark. Afterwards 0.5% (v/v) of the pre-culture was transferred to 50 mL of medium A with 4 % (w/v) of glucose. Afterwards, the cultivation continued for another 24 h under the same conditions as before. These cultures were then available as pre-cultures for cultivations in FlowerPlates in the Biolector cultivation system (m2p-labs GmbH, Baesweiler, Germany).

The composition of medium A is given in table 1.

**Table 1** Composition of medium A.

| Component | CAS Number | Amount per liter |
|---|---|---|
| $(NH_4)_2SO_4$ | 7783-20-2 | 20 g |
| Urea | 57-13-6 | 5 g |
| $KH_2PO_4$ | 7778-77-0 | 1 g |
| $K_2HPO_4$ | 7758-11-4 | 1 g |
| $MgSO_4$ * $7H_2O$ | 10034-99-8 | 0.25 g |
| 3-morpholinopropanesulfonic acid | 1132-61-2 | 42 g |
| $CaCl_2$ * $2H_2O$ | 10035-04-8 | 10 mg |
| $FeSO_4$ * $7H_2O$ | 7782-63-0 | 10 mg |
| $MnSO_4$ * $H_2O$ | 10034-96-5 | 10 mg |
| $ZnSO_4$ * $7H_2O$ | 7446-20-0 | 1 mg |
| $CuSO_4$ | 7758-98-7 | 0.2 mg |
| $NiCl_2$* $6H_2O$ | 7791-20-0 | 0.02 mg |
| Biotin (pH 7) | 58-85-5 | 0.2 mg |
| Glucose-monohydrate | 77938-63-7 | 44 g |
| Protocatechuic acid | 99-50-3 | 30 mg |

(continued)

| Component | CAS Number | Amount per liter |
|---|---|---|
| $H_2O$ | | ad 1000 mL |

**Example 2:** Response-surface methodology

Response-surface methodology (RSM) experiments were performed in the Biolector system. The FlowerPlates used contain 48 wells, which were filled with 980 µl of medium each. To inoculate, the pre-culture was centrifuged for 10 minutes at 4000 × g and resuspended in the medium A base, consisting only of urea, ammonium sulfate, di-potassium hydrogen phosphate and potassium di-hydrogen phosphate so that the final optical density at 600 nm was 50. 20 µl of this suspension were then used to inoculate each well of the FlowerPlate.

Multiple response surface experiments were performed. We designed the first to determine optimal concentrations for the main medium A components, i.e. urea, ammonium sulfate and both potassium phosphate salts as well as the carbon sources glucose and acetate. Building on preliminary results from this first set of experiments the effects of the trace components of the medium were then further tested, i.e. $MgSO_4$, $CaCl_2$, Biotin, PCA and the trace metal solution and subsequently the effects of the five separate trace metals contained in the solution, i.e. $FeSO_4$, $MnSO_4$, $ZnSO_4$, $CuSO_4$ and $NiCl_2$.

In general, all main media components were prepared as separate stock solutions at 10 × concentration (see Table 2). These were combined for the different media compositions to their desired concentrations and filled up to 1 mL with sterile water. As medium A contains both 1 g/L $KH_2PO_4$ and 1 g/L $K_2HPO_4$, we converted their total concentration to 13.05 mM phosphate. Trace elements were used at the following concentrations (see Table 3) for the first round of experiments.

The experimental design and analysis was done using R 3.6.1 (cf. R Core, T. A Language and Environment for Statistical Computing. 2019) and the rsm package version 2.10 (cf. Lenth, R. V. Response-Surface Methods in R, Using RSM. J. Stat. Softw. 2009, 32, 1-17, doi: 10.18637/jss.v032.i07).

The experimental design consisted of 24 different media compositions, performed in technical replicates. The concentration ranges for all compounds are shown in Table 2. These 48 experiments were done on one a single FlowerPlate, while center points and face-centered star points were done on a second plate, together with three repeated points of the first plate to allow comparison between the two and determination of block effects. The plates were then cultivated for 100 h at 30 °C and 1100 rpm. Optical densities were measured automatically every 15 minutes, as well as once at the end in the aforementioned photometer. At that time, also 0.5 mL of each well were subjected to carotenoid extraction and HPLC analysis.

The results of the response-surface model (RSM) were then analyzed and a set of new experiments based on the steepest ascent of astaxanthin concentration was performed in the FlowerPlates as already described.

Further experiments were then performed based on the preliminarily results of this first RSM and resulted in the medium composition CGAST1 consisting of 12.1 g/L potassium acetate, 42.6 g/L glucose, 19.3 g/L ammonium sulfate, 3.5 g/L urea and 3.8 g/L phosphate.

Concentrations of the trace elements were varied between 50% and 150% of normal medium A concentrations for the central design and 0% and 200% for the star points. Again, 24 different combinations were tested, although only as technical duplicates to allow for the simultaneous measurement of center points on every FlowerPlate. Again, final optical densities were measured and 0.5 mL of each well were harvested for carotenoid analysis.

Finally we prepared separate solutions of the trace metals, $FeSO_4$, $MnSO_4$, $ZnSO_4$, $CuSO_4$ and $NiCl_2$, contained in the previously used master mix and tested their effects by setting concentrations to 0% to 200% of their normal concentrations for the star points, 50% and 150% for the cube portion of the model and the normal medium A concentrations for the center points. These experiments were done in a single FlowerPlate and contained 7 center points, star points without replicates and the cube portion with 24 runs in the cube portion. The effect of nickel was confounded with the combined effect of the other four trace metals, thus leading to a fractional factorial design.

**Table 2** Overview of the 10 × stocks of the separately prepared media components for the design of experiment (DOE) approaches.

| Compound | Concentration | Notes | Final concentration range for DOE [g/L] | Sterilized by |
|---|---|---|---|---|
| Urea | 50 g/L | | 0 - 5 | Filtration |
| Ammonium sulfate | 200 g/L | | 10 - 30 | Autoclave |

(continued)

| Compound | Concentration | Notes | Final concentration range for DOE [g/L] | Sterilized by |
|---|---|---|---|---|
| $K_2HPO_4$<br>$KH_2PO_4$ | 2,273 g/L<br>1,776 g/L<br>Combined: 13,05 mM | Both solutions combined to a pH of 7 | 2 - 5 | Autoclave |
| MOPS | 420 g/L | Adjusted to pH 7 with KOH | 42 | Filtration |
| Glucose | 400 g/L | | 20 - 60 | Autoclave |
| Potassium acetate | 200 g/L | | 0 - 20 | Filtration |

**Table 3** Composition of medium A trace metal solution.

| Component | CAS Number | Amount per liter |
|---|---|---|
| $FeSO_4$ * $7H_2O$ | 7782-63-0 | 10 g/L |
| $MnSO_4$ * $H_2O$ | 10034-96-5 | 10 g/L |
| $ZnSO_4$* $7H_2O$ | 7446-20-0 | 1 g/L |
| $CuSO_4$ | 7758-98-7 | 0.2 g/L |
| $NiCl_2$ * $6H_2O$ | 7791-20-0 | 0.02 g/L |

**Example 3:** Extraction of carotenoids

To determine the carotenoid contents of the cells, 0.5 mL of culture were harvested for 10 minutes at 4000 X g. The supernatant was discarded and the cell pellet resuspended in 1 mL of methanol:acetone (7:3) and incubated for 30 minutes at 60 °C. Afterwards, the extract was separated from cell debris by centrifugation at 21.000 X g for 10 minutes and the supernatant collected for HPLC measurement. The extraction was repeated until the cell pellet was completely white, to achieve a complete removal of all carotenoids.

**Example 4:** HPLC analysis of carotenoids

The HPLC analysis was performed using an Agilent 1200 series system (Agilent Technologies, Waldbronn, Germany), equipped with a C18 reverse phase column (LiChrospher 100 RP18 EC-5, 125 $\times$ 4 mm (Merck KGaA, Darmstadt, Germany) and precolumn (LiChrospher 100 RP18 EC-5, 40 $\times$ 4 mm). Detection of the carotenoids was achieved using a diode array detector measuring and recording at wavelength 470 nm. Additionally, the detector recorded the whole spectrum between 250 nm and 500 nm. Authentic standards of astaxanthin, adonirubin, Canthaxanthincanthaxanthin, ß-carotene, ß-cryptoxanthin, echinenone and lycopene were measured to identify retention times and absorption coefficients for quantification.

Carotenoids were separated using a gradient between 9:1 methanol:water (A) and pure methanol (B) at 1.5 mL/min in the form of the following linear gradient: 0 min B: 0%, 10 min B: 100%, 32.5 min B: 100%, 33 min B: 0%.

**Example 5:** Optimization of main media components for carotenoid production

In order to identify important media components and to increase astaxanthin titers of *Corynebacterium glutamicum* strain ASTA*, a design of experiment (DOE) approach was followed. The main components of medium A ammonium sulfate, urea, phosphate and additionally the carbon sources glucose and potassium acetate were varied in their concentrations in the DOE.

The concentrations for the media components given in the following refer to the respective substances indicated in tables 1 and 3.

Except for phosphate, all of these components showed a positive correlation with biomass formation, although a negative two-factor interaction was identified between acetate and urea. Phosphate showed no influence on the optical density in the range analysed (see Table 4 and Figure 1).

**Table 4** Overview over the effects of main media components on cell dry weight and astaxanthin titers. The effect of each factor on the response, the corresponding F-value and the result of the ANOVA analysis are given as well. Factors separated by a colon indicate Two-factor interactions while squared factors indicate quadratic effects.

| Factor | F-value | Prob > F | F-value | Prob > F |
|---|---|---|---|---|
| Intercept | 7.01 | <0.001 | 13.85 | <0.001 |
| Block | -4.80 | <0.001 | -8.11 | <0.001 |
| Acetate | 6.84 | <0.001 | 14.50 | <0.001 |
| Glucose | 5.02 | <0.001 | 10.59 | <0.001 |
| Ammoniumsulphate | 0.87 | <0.001 | -1.28 | 0.21 |
| Urea | 2.57 | <0.001 | 14.30 | <0.001 |
| Phosphate | -0.72 | <0.001 | 5.20 | <0.001 |
| Acetate:Glucose | 3.37 | 0.00 | 6.13 | <0.001 |
| Acetate:Ammoniumsulphate | 1.56 | 0.12 | 0.55 | 0.59 |
| Acetate:Urea | -2.03 | 0.05 | -2.19 | 0.03 |
| Acetate: Phosphate | -0.79 | 0.43 | 2.33 | 0.02 |
| Glucose:Ammoniumsulphate | 3.23 | 0.00 | 0.10 | 0.92 |
| Glucose:Urea | 3.49 | <0.001 | 7.45 | <0.001 |
| Glucose:Phosphate | -2.17 | 0.03 | 0.10 | 0.92 |
| Ammoniumsulphate:Urea | 0.59 | 0.56 | 1.44 | 0.15 |
| Ammoniumsulphate:Phosphate | -1.89 | 0.06 | -0.03 | 0.97 |
| Urea:Phosphate | 1.51 | 0.14 | -0.94 | 0.35 |
| $Acetate^2$ | -0.60 | 0.55 | -6.24 | <0.001 |
| $Glucose^2$ | -0.58 | 0.56 | -9.19 | <0.001 |
| $Ammoniumsulphate^2$ | -0.26 | 0.80 | 0.31 | 0.76 |
| $Urea^2$ | -0.47 | 0.64 | -1.53 | 0.13 |
| $Phosphate^2$ | 0.07 | 0.94 | 0.64 | 0.53 |
| | F-value | Prob > F | F-value | Prob > F |
| Lack of fit | 2.36 | 0.04 | 3.07 | 0.01 |
| First order | 15.07 | <0.001 | 118.37 | <0.001 |
| Second order | 5.21 | <0.001 | 9.40 | <0.001 |
| Quadratic terms | 0.35 | 0.88 | 36.02 | <0.001 |

In regards to the astaxanthin titers, all main factors except for Ammonium sulfate had a positive influence. For both responses, the second-order model had a non-significant lack-of-fit and significant model terms.

Further experiments were done with the medium optimized for astaxanthin production. Both a steepest ascent and a stationary point analysis were performed as the stationary point identified at 12.7 g/L potassium acetate, 50 g/L glucose, 9.6 g/L ammonium sulfate, 7.4 g/L urea and 1.9 g/L phosphate was only a saddle point. The steepest ascent resulted in a medium composition of 12.1 g/L potassium acetate, 42.6 g/L glucose, 19.3 g/L ammonium sulfate, 3.5 g/L urea and 3.8 g/L phosphate. These two mediums were named medium B and medium C, respectively.

With these media it was possible to increase astaxanthin titers 2.1 and 3.2 fold, respectively, from 5.6 $\pm$ 0.4 mg/L to 12.1 $\pm$ 0.4 mg/L and 18,1 $\pm$ 4.2 mg/L (see Figure 1). The corresponding contour plots are shown in Figure 1. Furthermore, a clear increase in the total amount of carotenoids produced was visible as they increased from 36.8 $\pm$ 3.2 mg/L in medium A to 56.8 $\pm$ 8.1 mg/L and 89.3 $\pm$ 8.9 mg/L in medium B and medium C, respectively.

**Example 6:** Trace element optimization for carotenoid production

Based on medium B, a second round of Biolector experiments was performed to determine optima for further media components, specifically the medium A trace metal solution (see Table 3). The trace metal solution had a positive effect on astaxanthin production. Additionally, it was possible to increase the final cell dry weight (CDW) by increasing the $MgSO_4$ concentration. In order to further increase astaxanthin production and possibly also the production of further carotenoid species, the concentrations of these components at their medium A defaults and to focus was put on the trace metal solution.

The concentrations for the respective trace metals given in the following refer to the respective salts as indicated

in tables 1 and 3.

Since the medium A trace metal solution contains five different metal salts another experiment was performed to identify the singular effects of these metals. The resulting RSM is shown in Table 5 and the corresponding contour plots are shown in Figure 2. This experiment showed significant effects from $FeSO_4$, $MnSO_4$ and $ZnSO_4$. However, there was a significant negative interaction between iron and manganese, where high titers of manganese diminish the positive effect from high iron concentrations.

The identified optima for zinc and nickel were well within the experimental space and were thus set to 1.18 mg/L and 0,144 mg/L, respectively.

A second experiment was performed to further analyze astaxanthin production under a more limited set of trace metal concentrations. Zinc and nickel were set to their optimal concentrations while iron was tested between 11.68 mg/L and 28.32 mg/L, manganese between 0.84 mg/L and 9.16 mg/L and copper between 0.18 mg/L and 0.017 mg/L. In this experiment a further improvement was found at the new center point at an iron concentration of 20 mg/L, manganese concentration of 5 mg/L and copper concentration of 0.1 mg/L resulting in an astaxanthin titer of 35.9 ± 0.0 mg/L.

These trace metal solution was then combined with the previously established medium compositions medium B and medium C which resulted in a titer of 35.0 ± 1.1 mg/L in medium C and 33.6 ± 1.2 mg/L in medium B. While the titers were not significantly different, the total amount of carotenoids was significantly reduced in medium C to 54.2 ± 3.9 mg/L from 83.8 ± 2 mg/L in medium B leading to a potentially higher purity of isolated astaxanthin but also to a lower pool of carotenoids that can be converted to astaxanthin by future strain engineering (see Figure 3).

**Table 5** RSM for the effects of various trace metals on astaxanthin production and CDW in Corynebacterium glutamicum ASTA*.

| Factor | F-value | Prob > F | F-value | Prob > F |
|---|---|---|---|---|
| Intercept | 7.01 | <0.001 | 38.02 | <0.001 |
| $FeSO_4$ | 6.84 | <0.001 | 14.32 | <0.001 |
| $MnSO_4$ | 5.02 | <0.001 | -6.54 | <0.001 |
| $ZnSO_4$ | 0.87 | <0.001 | 5.13 | <0.001 |
| $CuSO_4$ | 2.57 | <0.001 | -1.00 | 0.33 |
| $NiCl_2$ | -0.72 | <0.001 | -2.06 | 0.05 |
| $FeSO_4$ : $MnSO_4$ | 3.37 | 0.00 | -2.32 | 0.03 |
| $FeSO_4$ : $ZnSO_4$ | 1.56 | 0.12 | -0.14 | 0.89 |
| $FeSO_4$ : $CuSO_4$ | -2.03 | 0.05 | 0.62 | 0.55 |
| $FeSO_4$ : $NiCl_2$ | -0.79 | 0.43 | 1.14 | 0.27 |
| $MnSO_4$ : $ZnSO_4$ | 3.23 | 0.00 | 0.10 | 0.92 |
| $MnSO_4$ : $CuSO_4$ | 3.49 | <0.001 | 1.49 | 0.15 |
| $MnSO_4$ : $NiCl_2$ | -2.17 | 0.03 | 0.34 | 0.74 |
| $ZnSO_4$ : $CuSO_4$ | 0.59 | 0.56 | -0.26 | 0.80 |
| $ZnSO_4$ : $NiCl_2$ | -1.89 | 0.06 | -0.51 | 0.61 |
| CuSO4 : $NiCl_2$ | 1.51 | 0.14 | 1.02 | 0.32 |
| $FeSO_4{}^2$ | -0.60 | 0.55 | -7.01 | <0.001 |
| $MnSO_4{}^2$ | -0.58 | 0.56 | -0.38 | 0.71 |
| $ZnSO_4{}^2$ | -0.26 | 0.80 | -8.19 | <0.001 |
| $CuSO_4{}^2$ | -0.47 | 0.64 | -0.86 | 0.40 |
| $NiCl_2{}^2$ | 0.07 | 0.94 | -2.08 | 0.05 |
| | F-value | Prob > F | F-value | Prob > F |
| Lack of fit | 2.36 | 0.04 | 5.16 | 0.01 |
| First order | 15.07 | <0.001 | 55.88 | <0.001 |
| Second order | 5.21 | <0.001 | 1.08 | 0.43 |
| Quadratic terms | 0.35 | 0.88 | 26.08 | <0.001 |

## Claims

1. Method for producing one or more species of carotenoids via one or more producer organism(s) comprising the steps

of

(i) Providing a growth medium comprising ammonium sulfate, urea, phosphate, glucose, and acetate,

wherein the concentration ratio of glucose to potassium acetate in said medium is in the range of from 3.0 to 4.0 and
the concentration ratio of glucose to ammonium sulfate in said medium is in the range of from 2.0 to 5.5 and
the concentration ratio of glucose to urea in said medium is in the range of from 6.0 to 12.5 and
the concentration ratio of glucose to phosphate in said medium is in the range of from 11.0 to 27.0;

(ii) Adding one or more producer organism(s) to said growth medium;
(iii) Cultivating said one or more producer organism(s) in said growth medium, under conditions allowing production of one or more species of carotenoids;
(iv) Optionally: extracting the one or more species of carotenoids.

**2.** Method according to claim 1, wherein the, one, two, three or more or all species of carotenoids is/are selected from the group consisting of astaxanthin, lycopene, β-carotene, echinenone, canthaxanthin, cryptoxanthin, hydroxyechinenone, zeaxanthin, adonirubin, and decaprenoxanthin, preferably astaxanthin, decaprenoxanthin, β-carotene, and canthaxanthin, particularly preferably astaxanthin.

**3.** Method according to any one of claims 1 or 2, wherein the, one, two, three or more or all producer organism(s) added in step (ii) is/are one or more microbial producer organism(s), preferably one or more bacterial producer organism(s), preferably selected from the phylum *Actinomycetota*, preferably selected from the class *Actinomycetia*, preferably selected from the order *Mycobacteriales*, preferably selected from the family *Corynebacteriaceae*, preferably selected from the genus *Corynebacterium,* particularly preferably the species *Corynebacterium glutamicum.*

**4.** Method according to any one of claims 1 to 3, wherein the, one, two, three or more or all producer organism(s) added in step (ii) comprise(s) an exogeneously expressed lycopene cyclase enzyme CrtY and/or an exogeneously expressed β-carotene ketolase enzyme CrtW and/or an exogenously expressed β-carotene hydroxylase enzyme CrtZ,

wherein the exogenously expressed lycopene cyclase enzyme CrtY has an amino acid sequence according to SEQ ID NO: 1 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, sequence similarity to the amino acid sequence according to SEQ ID No: 1,
and/or
wherein the exogenously expressed β-carotene ketolase enzyme CrtW has an amino acid sequence according to SEQ ID NO: 2 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, sequence similarity to the amino acid sequence according to SEQ ID No: 2,
and/or
wherein the exogenously expressed β-carotene hydroxylase enzyme CrtZ has an amino acid sequence according to SEQ ID NO: 3 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, sequence similarity to the amino acid sequence according to SEQ ID No: 3.

**5.** Method according to any one of claims 1 to 4, wherein the, one, two, three or more or all producer organism(s) added in step (ii) comprise(s) an exogenously expressed lycopene cyclase gene *crtY* and/or an exogenously expressed β-carotene ketolase gene *crtW* and/or an exogenously expressed β-carotene hydroxylase gene *crtZ*,

wherein the exogeneously expressed lycopene cyclase gene *crtY* has a nucleotide sequence according to SEQ ID NO: 4 or a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 4,
and/or
wherein the exogeneously expressed β-carotene ketolase gene *crrW* has a nucleotide sequence according to SEQ ID NO: 5 or a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 5,
and/or
wherein the exogenously expressed β-carotene hydroxylase gene *crtZ* has a nucleotide sequence according to SEQ ID NO: 6 or a nucleotide sequence having at least 70%, preferably at least 71%, preferably at least 72%, preferably at least 73%, preferably at least 74%, preferably at least 75%, preferably at least 76%, preferably at least 77%, preferably at least 78%, preferably at least 79%, preferably at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the nucleotide sequence according to SEQ ID No: 6.

**6.** Method according to any one of claims 1 to 5, wherein the growth medium provided in step (i) additionally comprises one or more substances suitable for allowing selection of the one or more producer organism(s) based on one or more reporter gene(s), preferably one or more selectable marker(s).

**7.** Method according to any one of claims 1 to 6, wherein the growth medium provided in step (i) additionally comprises one or more components selected from the group consisting of magnesium sulfate ($MgSO_4$), calcium chloride ($CaCl_2$), biotin, protocatechuic acid, iron sulfate ($FeSO_4$), manganese sulfate ($MnSO_4$), zinc sulfate ($ZnSO_4$), copper sulfate ($CuSO_4$), and nickel chloride ($NiCl_2$), preferably iron sulfate ($FeSO_4$), manganese sulfate ($MnSO_4$), zinc sulfate ($ZnSO_4$), copper sulfate ($CuSO_4$), and nickel chloride ($NiCl_2$), preferably iron sulfate ($FeSO_4$), manganese sulfate ($MnSO_4$), and zinc sulfate ($ZnSO_4$), particularly preferably iron sulfate ($FeSO_4$) and manganese sulfate ($MnSO_4$).

**8.** Method according to any one of claims 1 to 7, wherein the growth medium provided in step (i) comprises zinc at a concentration in the range of from 0.1 to 0.4 mg/L, preferably of from 0.15 to 0.35 mg/L, preferably of from 0.2 to 0.3 mg/L, preferably of from 0.25 to 0.28 mg/L particularly preferably of from 0.26 to 0.27 mg/L, and nickel at a concentration in the range of from 0.02 to 0.05 mg/L, preferably of from 0.025 to 0.04 mg/L, preferably of from 0.03 to 0.038 mg/L particularly preferably of from 0.035 to 0.036 mg/L, and iron at a concentration in the range of from 3.0 to 5.0 mg/L, preferably of from 3.5 to 4.5 mg/L, particularly preferably of from 4.0 to 4.1 mg/L, and manganese at a concentration in the range of from 1.4 to 2.0 mg/L, preferably of from 1.5 to 1.8 mg/L, particularly preferably of from 1.6 to 1.7 mg/L, and copper at a concentration in the range of from 0.0015 to 0.1 mg/L, preferably of from 0.02 to 0.05 mg/L, preferably of from 0.03 to 0.045 mg/L, particularly preferably of from 0.039 to 0.04 mg/L.

**9.** Method according to any one of claims 1 to 8, wherein the or, respectively, one of the one or more producer organism(s) is a strain of *Corynebacterium glutamicum* or a naturally occurring or artificially generated mutant thereof,
preferably wherein said strain of *Corynebacterium glutamicum* comprises at least one 16S rRNA molecule, which is encoded by a DNA sequence according to SEQ ID No: 16 or a DNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably

at least 96%, preferably at least 97%, preferably at least 98%, preferably at least 99% sequence identity to the DNA sequence according to SEQ ID No: 16.

**10.** Method according to any one of claims 1 to 9, wherein the or, respectively, one of the one or more producer organism(s) is a strain of *Corynebacterium glutamicum* or a naturally occurring or artificially generated mutant thereof, preferably wherein said strain of *Corynebacterium glutamicum* comprises at least one 16S rRNA molecule, which has an RNA sequence according to SEQ ID No: 17 or an RNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the RNA sequence according to SEQ ID No: 17.

**11.** Method according to any one of claims 1 to 10, wherein the growth medium provided in step (i) additionally comprises one or more solidifying agent(s), preferably selected from the group consisting of agar, agarose, alginate, and carrageenan, preferably agar.

**12.** Growth medium comprising ammonium sulfate, urea, phosphate, glucose, and potassium acetate, for production of one or more species of carotenoids via a producer organism,

wherein the concentration ratio of glucose to potassium acetate in said composition is in the range of from 3.0 to 4.0 and
the concentration ratio of glucose to ammonium sulfate in said composition is in the range of from 2.0 to 5.5 and
the concentration ratio of glucose to urea in said composition is in the range of from 6.0 to 12.5 and
the concentration ratio of glucose to phosphate in said composition is the range of from 11.0 to 27.0,

preferably wherein the growth medium is a growth medium as defined in any one of claims 6, 7, or 8.

**13.** Kit comprising the following components:

(a) Growth medium comprising ammonium sulfate, urea, phosphate, glucose, and potassium acetate, for production of one or more species of carotenoids via a producer organism,

wherein the concentration ratio of glucose to potassium acetate in said medium is in the range of from 3.0 to 4.0 and
the concentration ratio of glucose to ammonium sulfate in said medium is in the range of from 2.0 to 5.5 and
the concentration ratio of glucose to urea in said medium is in the range of from 6.0 to 12.5 and
the concentration ratio of glucose to phosphate in said medium is the range of from 11.0 to 27.0,
preferably wherein the growth medium is a growth medium as defined in any one of claims 6, 7, 8, or 10;

(b) One or more producer organism(s), preferably one or more bacterial producer organism(s), particularly preferably one or more producer organism(s) as defined in any one of claims 3, 4, 5, 9, or 10;
(c) optionally: One or more cultivation device(s) for cultivating said one or more producer organism(s) in said growth medium
(d) optionally: One or more pieces of laboratory consumables, preferably selected from the group consisting of disposable pipettes, disposable beakers, disposable flasks, disposable bottles, disposable tubes, chromatography columns, filters, spatulas, spoons;
(e) optionally: One or more pieces of laboratory equipment, preferably selected from the group consisting of glass pipettes, beakers, flasks, bottles, mixers, scales, incubators, measuring cylinders;
(f) optionally: Instructions for use.

**14.** Use of an expression construct or a vector in a method according to any one of claims 1 to 11, wherein the expression construct facilitates production of one or more species of carotenoids, preferably astaxanthin, lycopene, echinenone, cryptoxanthin, hydroxyechinenone, zeaxanthin, and adonirubin, preferably astaxanthin, β-carotene, and canthaxanthin, particularly preferably astaxanthin,
wherein the expression constructs encodes at least one lycopene cyclase enzyme CrtY and/or a β-carotene ketolase enzyme CrtW and/or at least one β-carotene hydroxylase enzyme CrtZ,

wherein the lycopene cyclase enzyme CrtY has an amino acid sequence according to SEQ ID NO: 1 or an amino

acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence similarity to the amino acid sequence according to SEQ ID No: 1,

and/or

wherein the β-carotene ketolase enzyme CrtW has an amino acid sequence according to SEQ ID NO: 2 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence similarity to the amino acid sequence according to SEQ ID No: 2,

and/or

wherein the β-carotene hydroxylase enzyme CrtZ has an amino acid sequence according to SEQ ID NO: 3 or an amino acid sequence having at least 80%, preferably at least 81%, preferably at least 82%, preferably at least 83%, preferably at least 84%, preferably at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence similarity to the amino acid sequence according to SEQ ID No: 3.

**15.** Use of a cell in a method according to any one of claims 1 to 11, wherein the cell comprises an expression construct according to claim 14, preferably wherein said cell is a *Corynebacterium glutamicum* cell and comprises at least one 16S rRNA molecule, which is encoded by a DNA sequence according to SEQ ID No: 16 or a DNA sequence having at least 85%, preferably at least 86%, preferably at least 87%, preferably at least 88%, preferably at least 89%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99% sequence identity to the DNA sequence according to SEQ ID No: 16.

**Figure 1**

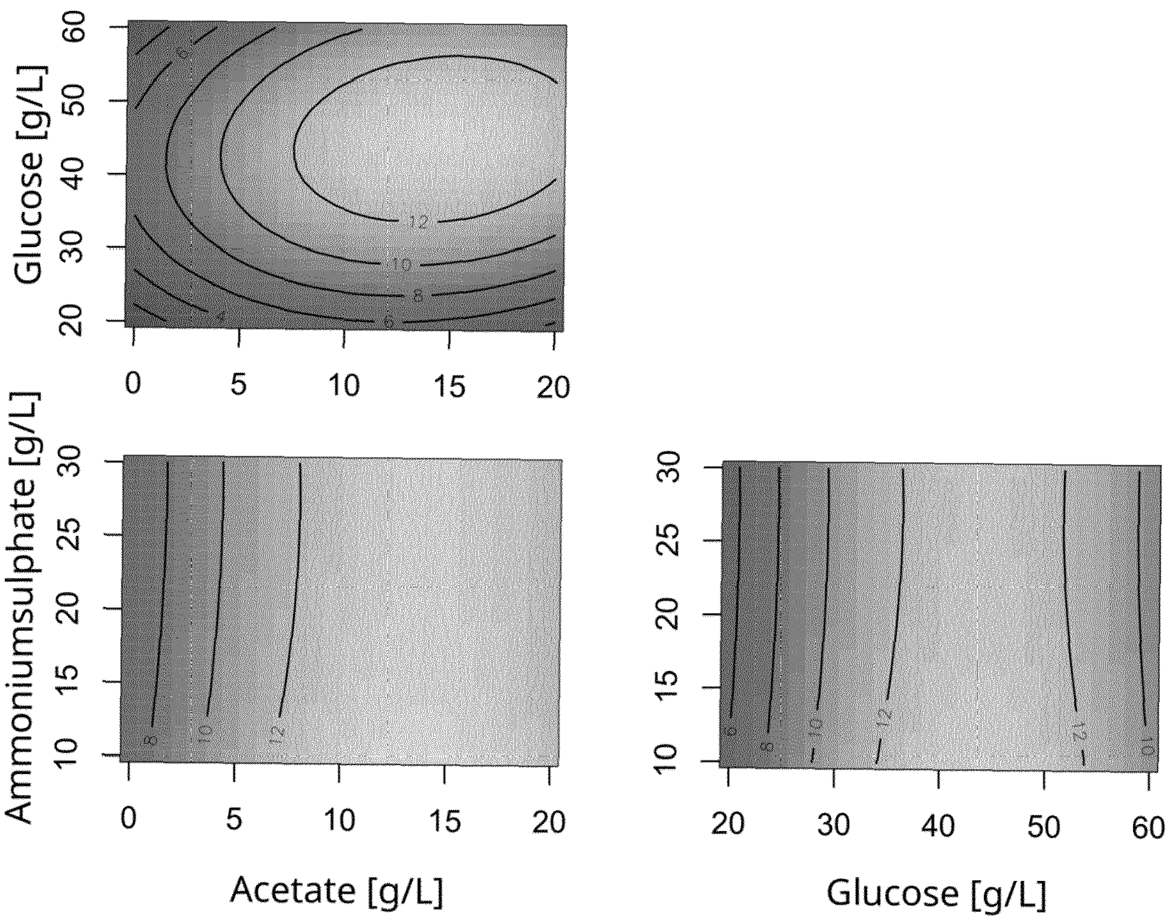

Figure 1 (continued)

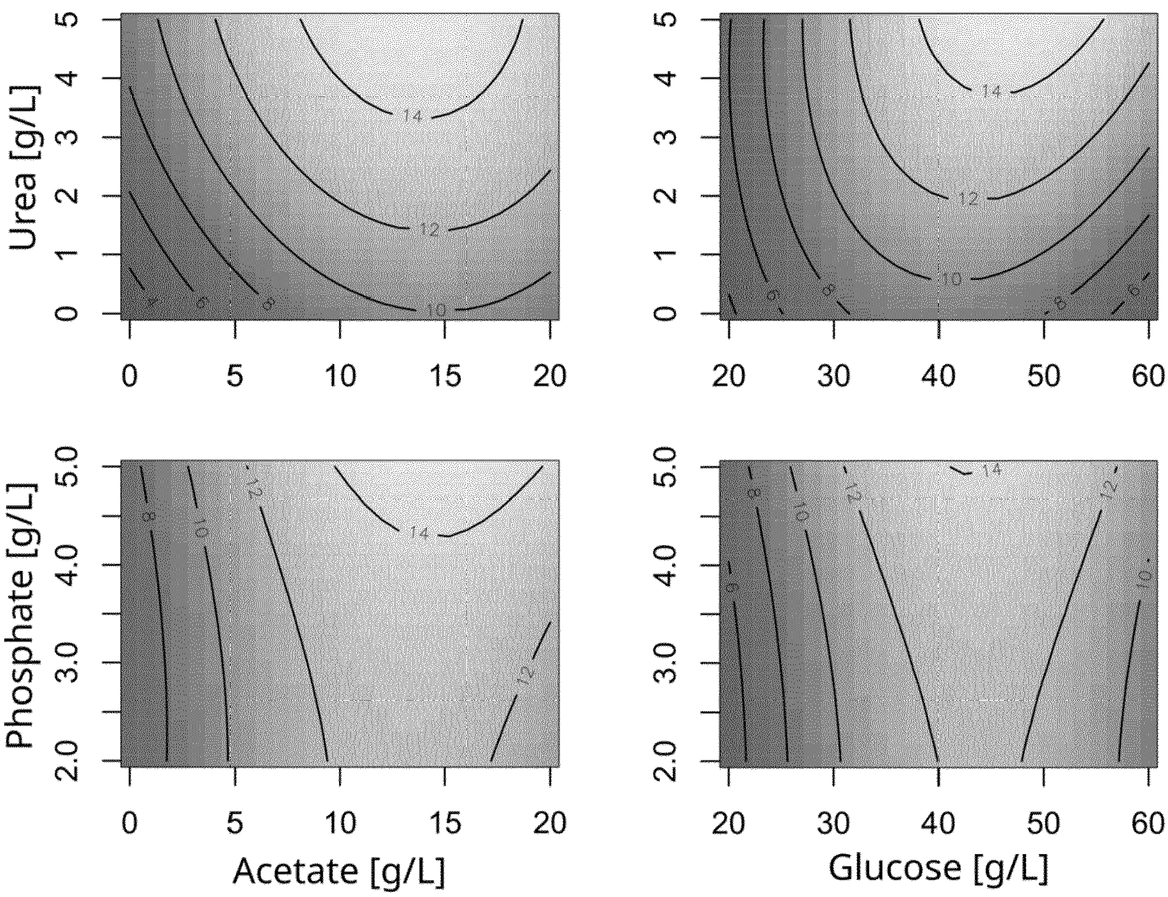

**Figure 1 (continued)**

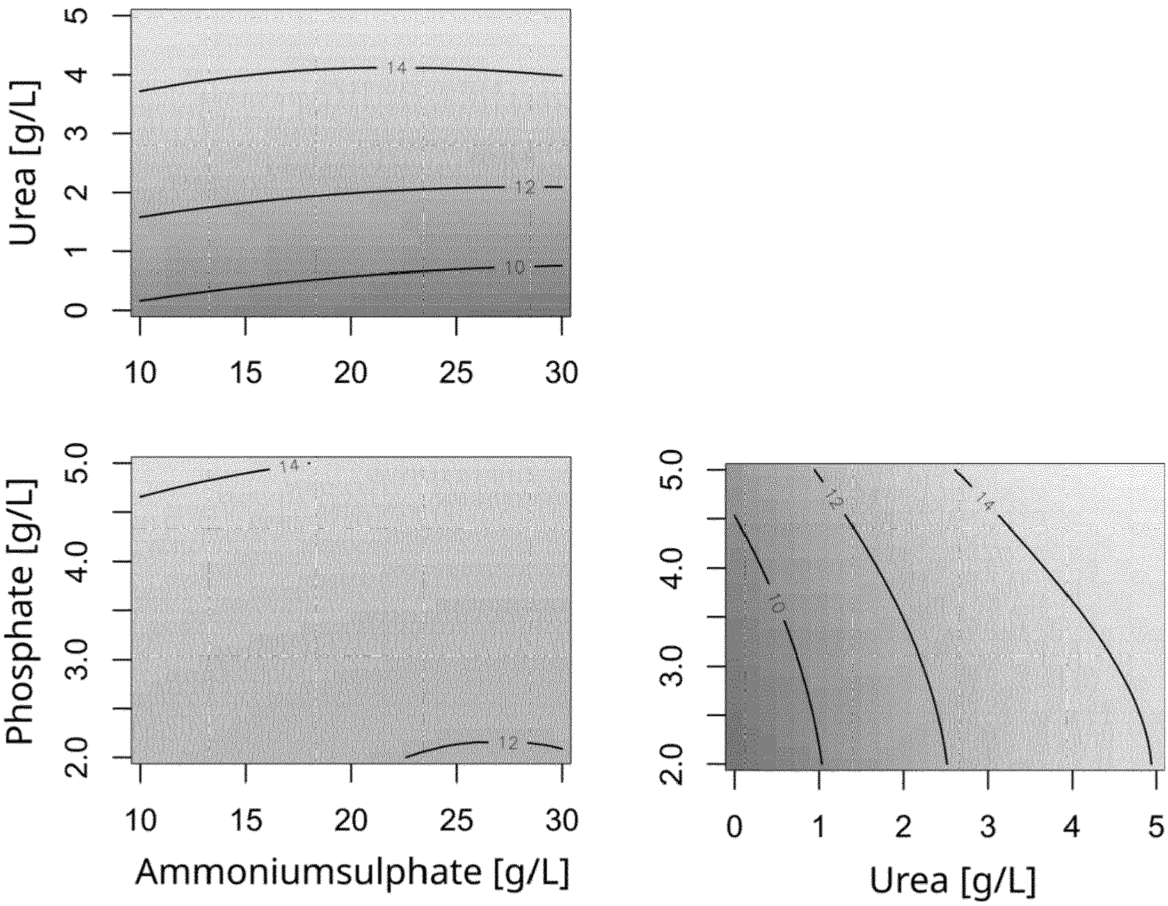

Figure 2

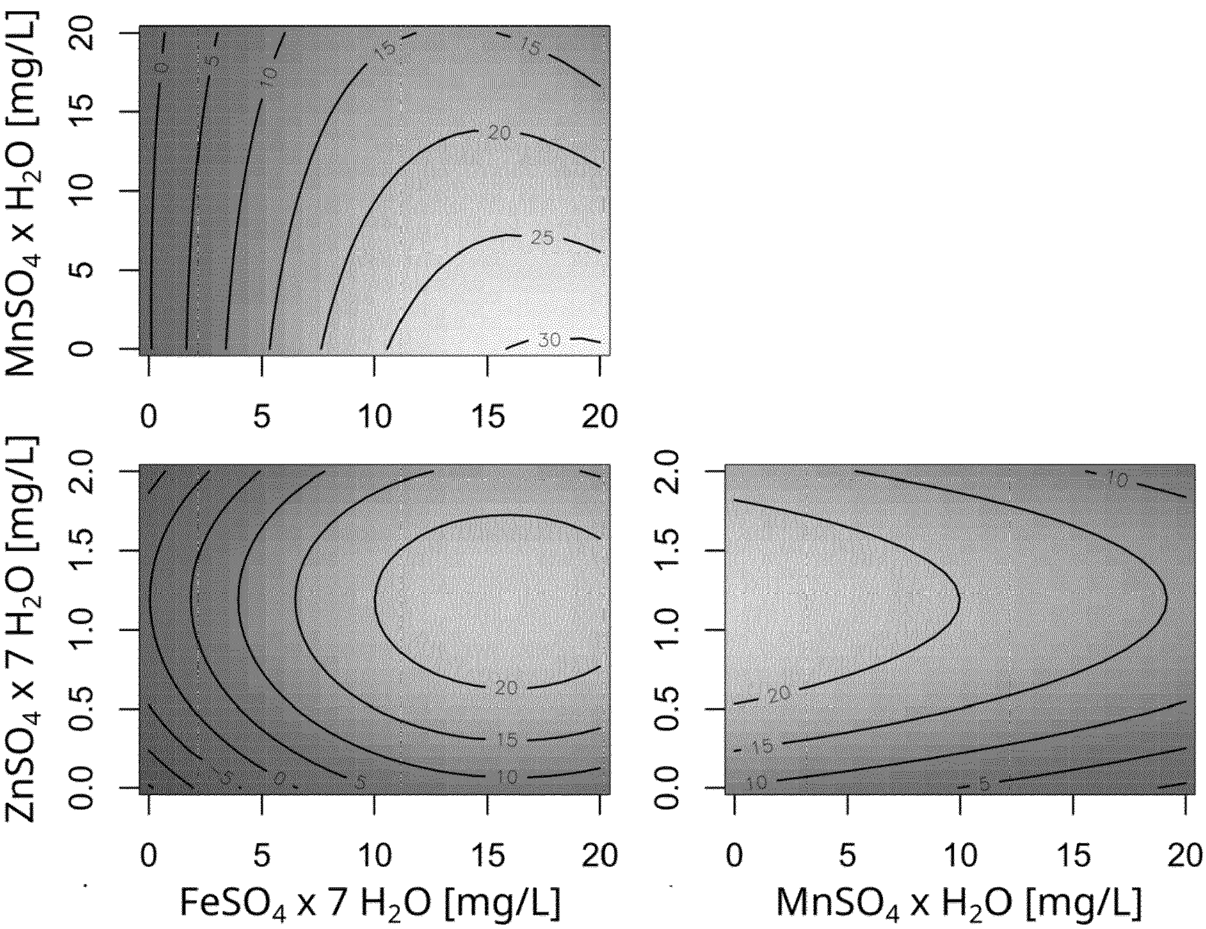
MnSO4 x H2O [mg/L]
ZnSO4 x 7 H2O [mg/L]
FeSO4 x 7 H2O [mg/L]
MnSO4 x H2O [mg/L]
0
5
10
15
20
25
30
0.0
0.5
1.0
1.5
2.0

Figure 2 (continued)

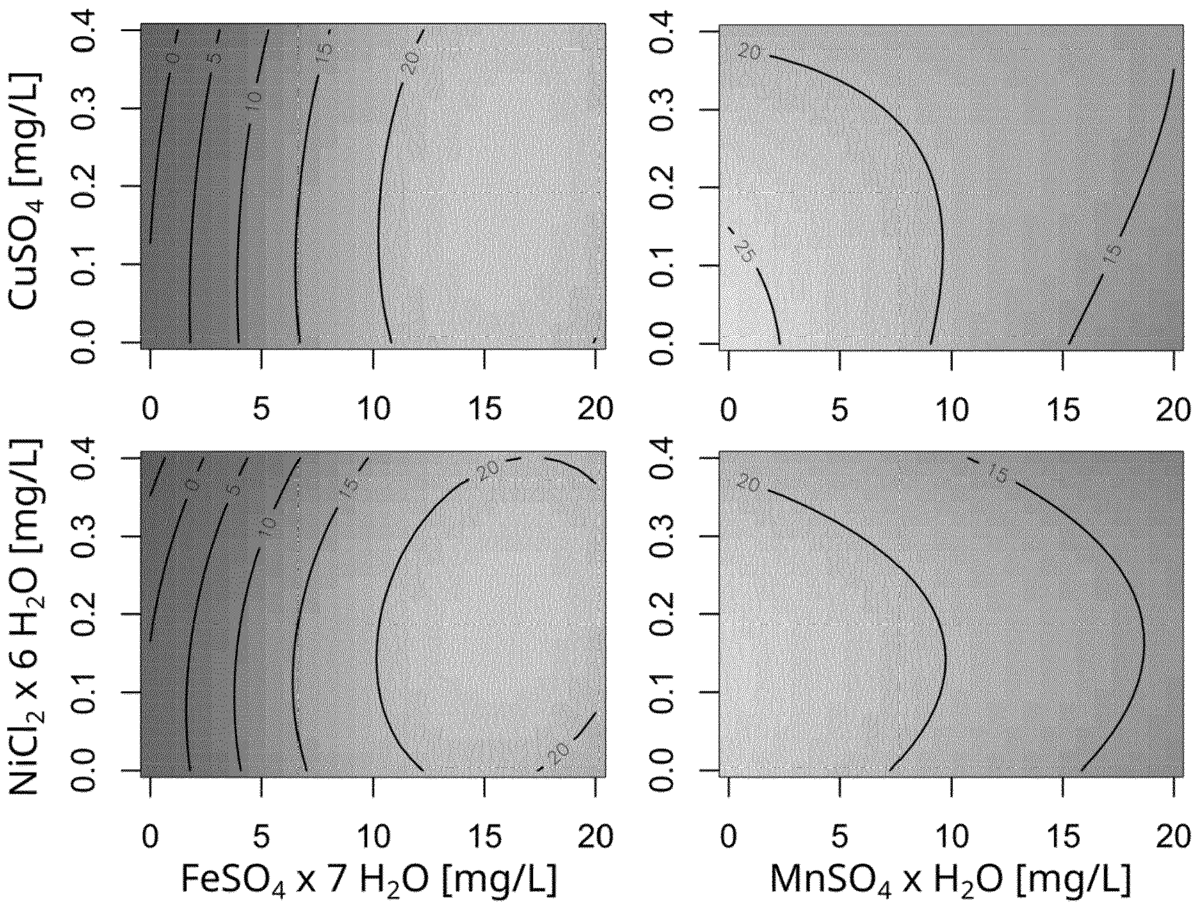

Figure 2 (continued)

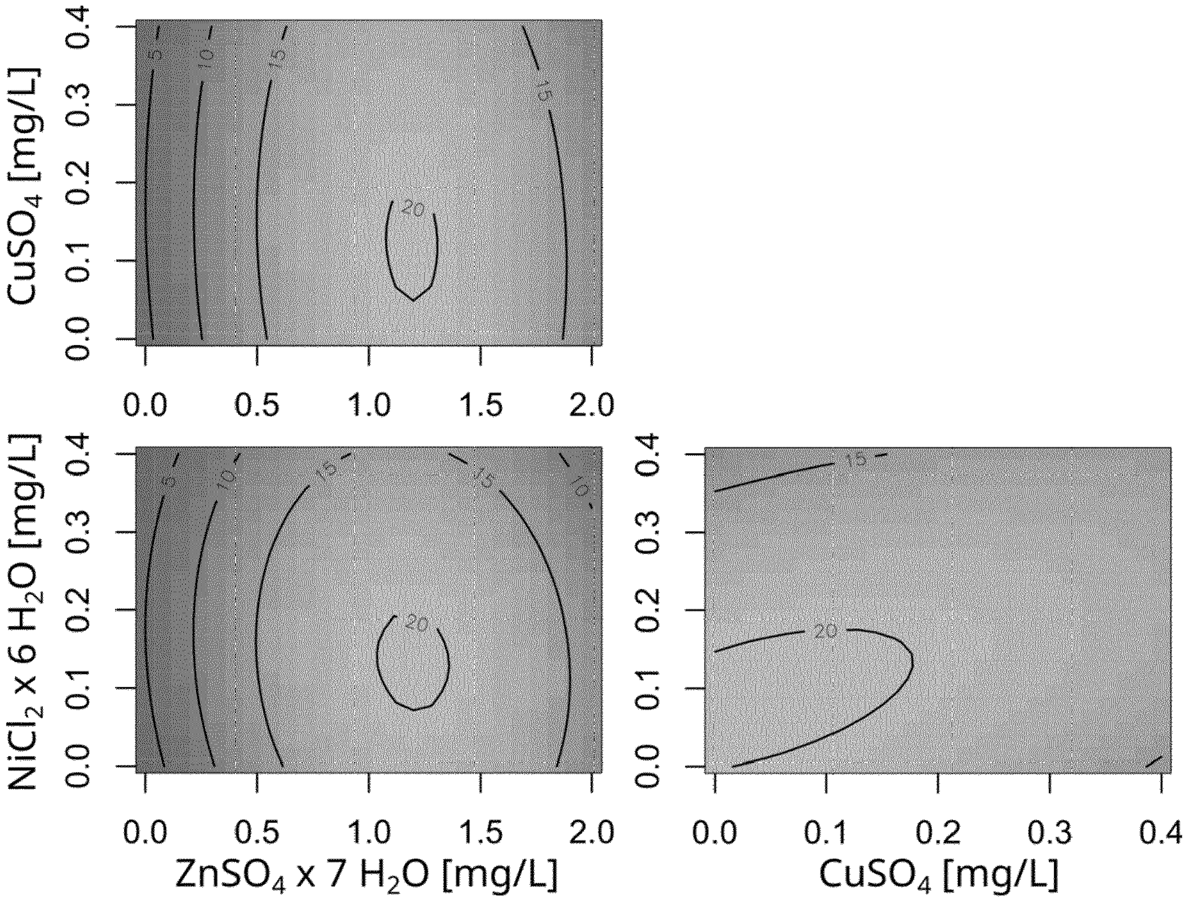

Figure 3

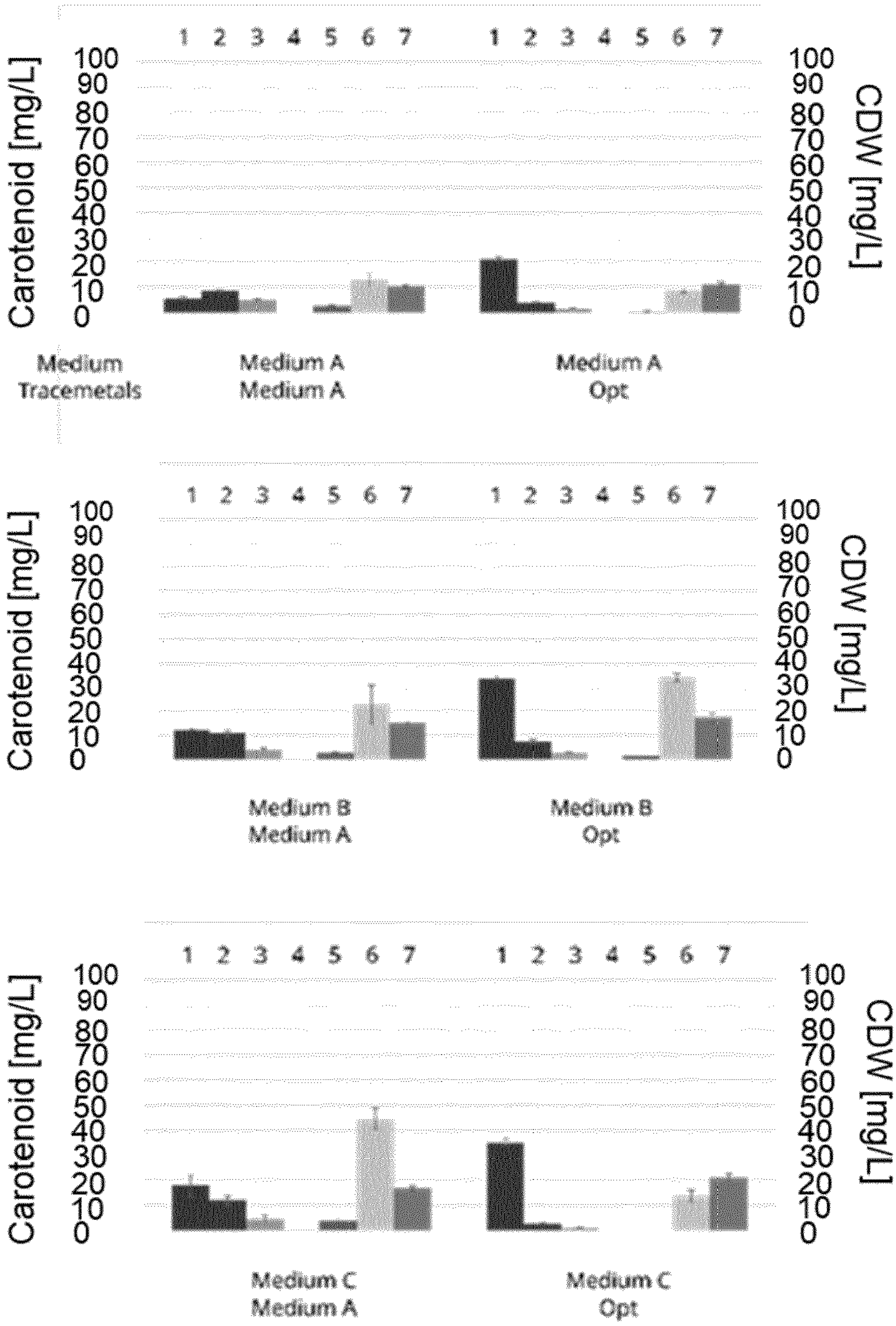
1 2 3 4 5 6 7 1 2 3 4 5 6 7
Carotenoid [mg/L]
CDW [mg/L]
100 90 80 70 60 50 40 30 20 10 0
Medium Tracemetals
Medium A Medium A
Medium A Opt
Medium B Medium A
Medium B Opt
Medium C Medium A
Medium C Opt

Figure 3 (continued)

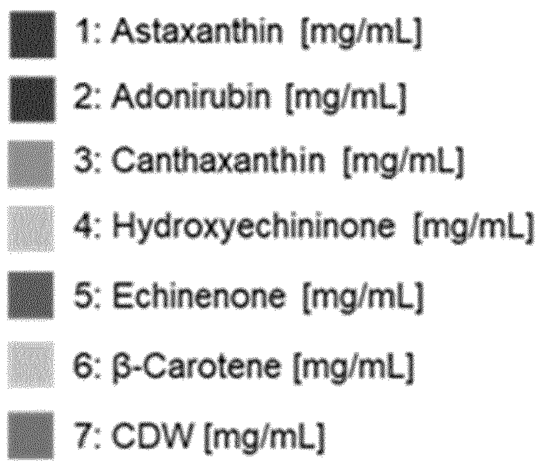

Europäisches Patentamt
European Patent Office
Office européen des brevets

# EUROPEAN SEARCH REPORT

Application Number
EP 23 20 2738

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | HENKE NADJA A. ET AL: "Improved Astaxanthin Production with Corynebacterium glutamicum by Application of a Membrane Fusion Protein", MARINE DRUGS, vol. 17, 621, 1 November 2019 (2019-11-01), pages 1-12, XP093144874, Basel, CH ISSN: 1660-3397, DOI: 10.3390/md17110621 * abstract; figure 1 * * paragraph [2.3.]; figure 5 * * page 7, last paragraph - page 8, paragraph 1 * * table 1 * ----- | 1-15 | INV. C12P5/00 C12N1/20 C12N9/02 C12N9/90 C12P23/00 |
| A | US 2020/181660 A1 (PETERS-WENDISCH PETRA [DE] ET AL) 11 June 2020 (2020-06-11) * abstract; claims; examples * * tables 2, 3 * ----- | 1-15 | |
| A | US 2019/161780 A1 (YAMADA KAZUTERU [JP] ET AL) 30 May 2019 (2019-05-30) * abstract; claims; examples * ----- | 1-15 | TECHNICAL FIELDS SEARCHED (IPC) C12P C12N C12R |
| A | US 2014/335576 A1 (CHOTANI GOPAL K [US] ET AL) 13 November 2014 (2014-11-13) * abstract; claims; examples * * paragraphs [0168], [0226], [0241], [0260] * ----- | 1-15 | |

The present search report has been drawn up for all claims

2

| Place of search | Date of completion of the search | Examiner |
|---|---|---|
| Berlin | 24 March 2024 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 20 2738

This annex lists the patent family members relating to the patent documents cited in the above-mentioned European search report. The members are as contained in the European Patent Office EDP file on
The European Patent Office is in no way liable for these particulars which are merely given for the purpose of information.

24-03-2024

| Patent document cited in search report | Publication date | Patent family member(s) | Publication date |
| --- | --- | --- | --- |
| US 2020181660 A1 | 11-06-2020 | EP 3395827 A1 | 31-10-2018 |
| | | EP 3615557 A1 | 04-03-2020 |
| | | US 2020181660 A1 | 11-06-2020 |
| | | WO 2018197608 A1 | 01-11-2018 |
| US 2019161780 A1 | 30-05-2019 | CA 3033334 A1 | 15-02-2018 |
| | | CL 2019000318 A1 | 07-06-2019 |
| | | EP 3498836 A1 | 19-06-2019 |
| | | JP 2019165635 A | 03-10-2019 |
| | | US 2019161780 A1 | 30-05-2019 |
| | | WO 2018030507 A1 | 15-02-2018 |
| US 2014335576 A1 | 13-11-2014 | CA 2850794 A1 | 11-04-2013 |
| | | EP 2764108 A2 | 13-08-2014 |
| | | US 2013273625 A1 | 17-10-2013 |
| | | US 2014335576 A1 | 13-11-2014 |
| | | WO 2013052914 A2 | 11-04-2013 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090221027 A
- WO 2018197608 A1


### Non-patent literature cited in the description


- **SMITH, T.F.** ; **WATERMAN, M.S.** identification of common molecular subsequences. *Journal of Molecular Biology*, 1981, vol. 147 (1), 195-197
- *CHEMICAL ABSTRACTS*, 7783-20-2
- *CHEMICAL ABSTRACTS*, 14808-79-8
- *CHEMICAL ABSTRACTS*, 57-13-6
- *CHEMICAL ABSTRACTS*, 14265-44-2
- *CHEMICAL ABSTRACTS*, 50-99-7
- *CHEMICAL ABSTRACTS*, 7487-88-9
- *CHEMICAL ABSTRACTS*, 14168-73-1
- *CHEMICAL ABSTRACTS*, 24378-31-2
- *CHEMICAL ABSTRACTS*, 15553-21-6
- *CHEMICAL ABSTRACTS*, 13778-97-7
- *CHEMICAL ABSTRACTS*, 10034-99-8
- *CHEMICAL ABSTRACTS*, 10043-52-4
- *CHEMICAL ABSTRACTS*, 22691-02-7
- *CHEMICAL ABSTRACTS*, 10035-04-8
- *CHEMICAL ABSTRACTS*, 25094-02-4
- *CHEMICAL ABSTRACTS*, 7774-34-7
- *CHEMICAL ABSTRACTS*, 58-85-5
- *CHEMICAL ABSTRACTS*, 99-50-3
- *CHEMICAL ABSTRACTS*, 7720-78-7
- *CHEMICAL ABSTRACTS*, 17375-41-6
- *CHEMICAL ABSTRACTS*, 10028-21-4
- *CHEMICAL ABSTRACTS*, 7782-63-0
- *CHEMICAL ABSTRACTS*, 7785-87-7
- *CHEMICAL ABSTRACTS*, 10034-96-5
- *CHEMICAL ABSTRACTS*, 10101-68-5
- *CHEMICAL ABSTRACTS*, 7733-02-0
- *CHEMICAL ABSTRACTS*, 7446-19-7
- *CHEMICAL ABSTRACTS*, 13986-24-8
- *CHEMICAL ABSTRACTS*, 7446-20-0
- *CHEMICAL ABSTRACTS*, 7758-98-7
- *CHEMICAL ABSTRACTS*, 16448-28-5
- *CHEMICAL ABSTRACTS*, 19086-18-1
- *CHEMICAL ABSTRACTS*, 7718-54-9
- *CHEMICAL ABSTRACTS*, 7791-20-0
- **HENKE, N. A.** ; **WENDISCH, V. F.** Improved Astaxanthin Production with Corynebacterium Glutamicum by Application of a Membrane Fusion Protein. *Mar. Drugs*, 2019, vol. 17
- **R CORE, T. A**. *Language and Environment for Statistical Computing*, 2019
- **LENTH, R. V.** Response-Surface Methods in R, Using RSM. *J. Stat. Softw.*, 2009, vol. 32, 1-17